(19) Europäisches Patentamt

European Patent Office

Office européen des brevets

(11) **EP 4 553 156 A1**

(12) **EUROPEAN PATENT APPLICATION**

(43) Date of publication:
**14.05.2025  Bulletin 2025/20**

(21) Application number: **23208666.0**

(22) Date of filing: **08.11.2023**

(51) International Patent Classification (IPC):
*C12N 9/22* (2006.01)   *C12N 15/10* (2006.01)
*C12N 15/113* (2010.01)   *C12N 15/63* (2006.01)
*C12N 15/64* (2006.01)   *C12Q 1/00* (2006.01)
*C12Q 1/6806* (2018.01)

(52) Cooperative Patent Classification (CPC):
**C12N 9/22; C12N 15/66; C12Q 1/68; C12Q 1/6806;
C12Q 1/6855;** C12N 2310/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **4basebio UK Ltd
Cambridge CB24 5QE (GB)**

(72) Inventors:
• **LANCKRIET, Heikki**
  **Cambridge, CB24 5QE (GB)**
• **WALKER, Amy**
  **Cambridge, CB24 5QE (GB)**
• **BOUCHAREB, Amine**
  **Cambridge, CB24 5QE (GB)**
• **RISTIN, Anca-Paula**
  **Cambridge, CB24 5QE (GB)**

(74) Representative: **Boult Wade Tennant LLP
Salisbury Square House
8 Salisbury Square
London EC4Y 8AP (GB)**

(54) **SYNTHETIC DNA PRODUCTION USING CAS NUCLEASE**

(57)    The present invention relates to methods for producing a linear deoxyribonucleic acid (DNA) product (e.g. a closed linear DNA product) with enhanced resistance to nuclease digestion using a method comprising a cas nuclease. The present invention relates to methods comprising contacting a doublestranded DNA molecule with an RNP complex comprising a single stranded guide RNA (gRNA) and a cas nuclease, a ligase and first and second adaptor molecules to generate a linear DNA product. The step of contacting the DNA molecule with the RNP complex may be carried out prior to or simultaneously with the step of contacting with a ligase and first and second adaptor molecules.

**Description**

**TECHNICAL FIELD**

The present invention relates to methods for producing a linear deoxyribonucleic acid (DNA) product (e.g. a closed linear DNA product) with enhanced resistance to nuclease digestion using a method comprising a cas nuclease. The present invention relates to methods comprising contacting a double-stranded DNA molecule with an ribonucleoprotein (RNP) complex comprising a single stranded guide RNA (gRNA) and a cas nuclease, a ligase and first and second adaptor molecules to generate a linear DNA product. The step of contacting the DNA molecule with the RNP complex may be carried out prior to or simultaneously with the step of contacting with a ligase and first and second adaptor molecules.

**BACKGROUND**

The CRISPR-cas system is an adaptive immune system that exists in many bacteria and has been exploited in molecular biology research over the last decade or so. The use of this system allows for editing of a genome within a cell. Cas nucleases are able to cleave nucleic acid at a site where a guide RNA is complementary to the DNA - a gRNA. At the site of cleavage, the DNA is repaired by joining the two cut ends, or a new sequence may be inserted.

In particular, cpf1 is a class 2 CRISPR nuclease, which is a single-RNA guided endonuclease. It has a TTTN PAM (protospacer-adjacent motif), where N is A, G or C and cleaves DNA to produce overhangs, unlike the blunt ends produced by cas9, the most-used cas nuclease in gene editing. The overhangs produced may be 4 or 5 nucleotides in length.

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

For linear molecules, one of the proposed solutions includes the use of phosphorothioated nucleotides (i.e. 2'-deoxynucleotides-5'-($\alpha$-thio)-triphosphate).

Alternatively, resistance to nuclease digestion may be accomplished by producing closed linear DNA molecules. For example, WO2010/086626 A1 describes a method for producing a closed linear DNA by utilizing a protelomerase. However, this method is limited in that the action of protelomerase produces the same sequence at both ends of the closed linear DNA molecule.

Alternative methods are described in WO2022/229460 and WO2023/006978. However, these methods rely on the presence of an endonuclease target sequence that is absent from the gene of interest, or cassette. Occasionally, particularly for a long cassette (sequence of interest) sequence, it may be difficult to find a unique endonuclease sequence for digesting the DNA molecule without cutting the cassette (sequence of interest) at the same time.

Thus, a need exists for a more flexible method for producing a linear DNA product with an enhanced resistance to nuclease (e.g. exonuclease) digestion.

**DESCRIPTION**

The invention provides a method for producing a linear deoxyribonucleic acid (DNA) product (e.g. a closed linear DNA product) with enhanced resistance to nuclease digestion. The invention is based on the digestion of a double-stranded DNA molecule using a cas nuclease and the addition of adaptor molecules. In one aspect, the method of the invention relies on the addition of the adaptor molecules, a ribonucleoprotein (hereafter, an RNP) complex, comprising at least one guide RNA (gRNA, as used herein) and a cas nuclease, and a ligase to the double-stranded DNA molecule in a single reaction volume (or single contiguous aqueous volume). Thus, the method for producing a linear DNA product comprises the steps: (a) contacting a double-stranded DNA molecule comprising at least one PAM sequence with an RNP complex comprising at least one gRNA and a cas nuclease, and a ligase and first and second adaptor molecules to form a single contiguous aqueous volume; and (b) incubating the single contiguous aqueous volume to generate the linear DNA product. Alternatively, the method for producing a linear deoxyribonucleic acid (DNA) product comprises: (a) contacting a double-stranded DNA molecule comprising at least one PAM sequence with an RNP complex comprising a guide RNA (gRNA) and a cas nuclease to form a digested DNA; and (b) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region. Preferably, the linear DNA product has enhanced resistance to exonuclease (e.g. exonuclease I, exonuclease III and/or exonuclease VIII) digestion. The linear DNA product may be a closed linear DNA product. The linear DNA product may comprise nuclease-resistant nucleotides (i.e. protected nucleotides), such as phosphorothioated nucleotides. The linear DNA product may be a partially closed linear DNA product. The partially closed linear DNA product may comprise nuclease-resistant nucleotides (i.e. protected nucleotides), such as phosphorothioated nucleotides. The linear DNA product may comprise a cassette. The cassette may comprise a coding sequence.

The invention provides a method for producing a linear DNA product, wherein the method comprises:

(a) contacting a double-stranded DNA molecule comprising at least one PAM sequence with an RNP complex comprising at least one gRNA and a cas nuclease, and first and second adaptor molecules to form a single contiguous aqueous volume; and
(b) incubating the single contiguous aqueous volume to generate the linear DNA product, wherein the linear DNA product comprises a linear double-stranded region, wherein the linear double-stranded region comprises a portion of the double-stranded DNA molecule, and wherein the first adaptor molecule is appended to a first end of the linear double-stranded region and the second adaptor molecule is appended to a second end of the linear double-stranded region.

Also provided is a method for producing a linear deoxyribonucleic acid (DNA) product, wherein the method comprises:

(a) contacting a double-stranded DNA molecule comprising at least one PAM sequence with an RNP complex comprising a single stranded guide RNA (gRNA) and a cas nuclease to form a digested DNA;
(b) contacting the digested DNA with first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence 5' of the target sequence.

The first and second adaptor molecules may be identical molecules or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. Thus, the linear DNA product produced by the methods described herein is resistant to nuclease (e.g. exonuclease) digestion.

The step of contacting the double-stranded DNA molecule with an RNP complex comprising at least one gRNA and a cas nuclease, and first and second adaptor molecules is preferably performed in the presence of a ligase. Thus, the invention provides a method for producing a linear deoxyribonucleic acid (DNA) product, wherein the method comprises:

(a) contacting a double-stranded DNA molecule comprising at least one PAM sequence with an RNP complex comprising at least one gRNA and a cas nuclease, a ligase and first and second adaptor molecules to form a single contiguous aqueous volume; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence.

In the presence of a ligase, the method may comprise:

(a) contacting a double-stranded DNA molecule comprising at least one PAM sequence with an RNP complex comprising a single stranded guide RNA (gRNA) and a cas nuclease to form a digested DNA;
(b) contacting the digested DNA with a ligase and first and second adaptor molecules to generate the linear DNA product, wherein the linear DNA product comprises a linear double-stranded region, and wherein the first adaptor molecule is ligated to a first end of the linear double-stranded region and the second adaptor molecule is ligated to a second end of the linear double-stranded region.

Preferably, the cas nuclease is cpf1. The step of contacting the DNA with the RNP complex may be carried out in

the presence of $Mg^{2+}$. The double stranded DNA may comprise a PAM sequence.

The appending (or linking or closing) of the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The appending of the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region. The appending may be performed via a linker or spacer molecule which facilitates joining of the adaptor molecule to the first and/or second end of the linear double-stranded region.

The method may further comprise, before step (a) (i.e. the step of contacting the double-stranded DNA molecule with the an RNP complex comprising at least one gRNA and a cas nuclease, the ligase and the first and second adaptor molecules), a step of amplification of a DNA template molecule to produce the double-stranded DNA molecule. Thus, the invention provides a method for producing a linear DNA product, the method comprises:

(a) amplification of a DNA template molecule comprising at least one target sequence and at least one PAM sequence to generate a double-stranded DNA molecule;
(b) contacting the double-stranded DNA molecule with an RNP complex comprising at least one gRNA and a cas nuclease, a ligase and first and second adaptor molecules to form a single contiguous aqueous volume, wherein the at least one gRNA is complementary to the at least one target sequence; and
(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence.

Alternatively, the method may comprise

(a) amplification of a DNA template molecule comprising at least one target sequence and at least one PAM sequence to generate a double-stranded DNA molecule
(b) contacting a double-stranded DNA molecule with an RNP complex comprising a single stranded guide RNA (gRNA) and a cas nuclease to form a digested DNA, wherein the gRNA has a sequence complementary to the target sequence;
(c) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence.

The amplification may be an in vitro or in vivo amplification. Preferably, the amplification is an in vitro amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification. Thus, the invention provides a method for producing a linear deoxyribonucleic acid (DNA) product, the method comprises:

(a) rolling circle amplification of a DNA template molecule comprising at least one target sequence and at least one PAM sequence to generate a double-stranded DNA molecule;
(b) contacting the double-stranded DNA molecule with an RNP complex comprising at least one gRNA and a cas nuclease, a ligase and first and second adaptor molecules to form a single contiguous aqueous volume, wherein the at least one gRNA is complementary to the at least one target sequence; and

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence 5' of the target sequence.

The method may further comprise (after the step of amplification and before the step of contacting the double-stranded DNA molecule with an RNP complex comprising at least one gRNA and a cas nuclease, a ligase and first and second adaptor molecules) a step of heat-deactivation. Thus, the invention provides a method for producing a linear DNA product, the method comprises:

(a) amplification of a DNA template molecule comprising at least one target sequence and at least one PAM sequence to generate a double-stranded DNA molecule;
(b) heat-deactivation of the reaction of step (a);
(c) contacting the double-stranded DNA molecule with an RNP complex comprising at least one gRNA and a cas nuclease, a ligase and first and second adaptor molecules to form a single contiguous aqueous volume, wherein the at least one gRNA has a sequence complementary to the at least one target sequence; and
(d) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence 5' of the target sequence. The PAM sequence may be present upstream of the gene of interest/ the cassette. The PAM sequence may be downstream of the gene of interest. A PAM sequence (and a target sequence) may be present both upstream and downstream of the gene or sequence of interest/cassette.

Alternatively, the method may comprise

(a) amplification of a DNA template molecule comprising at least one target sequence and at least one PAM sequence to generate a double-stranded DNA molecule;
(b) heat deactivation of the reaction of step (a)
(c) contacting the double-stranded DNA molecule with an RNP complex comprising a single stranded guide RNA (gRNA) and a cas nuclease to form a digested DNA, wherein the at least one gRNA has a sequence complementary to the at least one target sequence; and
(d) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region.

Preferably, the cas nuclease is cpf1. The step of contacting the DNA with the RNP complex may be carried out in the presence of $Mg^{2+}$. The double stranded DNA may comprise a PAM sequence 5' of the target sequence. The PAM sequence may be present upstream of the gene of interest/the cassette. The PAM sequence may be downstream of the gene of interest. A PAM sequence (and a target sequence) may be present both upstream and downstream of the gene or sequence of interest/cassette.

Preferably, amplification is rolling-circle amplification.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors of the present application have surprisingly discovered that large concatemeric products of the rolling circle amplification reaction can be used to produce the DNA products described herein. This is surprising as the product of the rolling circle amplification has high viscosity and typically has to undergo purification steps before it can be utilized for downstream applications.

In the method described herein, after the step of amplification, the step of contacting the double-stranded DNA molecule with an endonuclease, a ligase and first and second adaptor molecules to form a single contiguous aqueous volume may be performed without purifying the product of the amplification reaction. That is to say that the step of contacting the double-stranded DNA molecule with an endonuclease, a ligase and first and second adaptor molecules may be performed directly after the step of amplification. The step of contacting the double-stranded DNA molecule with an endonuclease, a ligase and first and second adaptor molecules may be performed directly after the step of heat-deactivation.

The inventors of the present application have discovered a method which requires very few steps to produce the DNA product described herein. The methods described herein are very time efficient. This is, in part, due to the fact that a step of purification is not required after the amplification reaction. Optionally, the product of the amplification reaction may be heat-deactivated. Surprising, the method described herein where the step or steps of contacting the double-stranded DNA molecule with an RNP complex comprising a cas nuclease and at least one gRNA, and a ligase and first and second adaptor molecules is performed directly after the step of amplification (i.e. without the step of purification), produces higher yields of the DNA product described herein when compared to a method in which the two steps are separated by purification of the amplification product.

The use of a cas nuclease, preferably cpf1, allows any sequence to be cleaved, since a guide RNA (gRNA) can be designed to target any sequence. Alternatively, the sequence of interest, or cassette may be cloned into a universal vector, such that the same gRNA can be used for the digestion and ligation of adaptor molecules to different cassettes or genes or sequences of interest, to produce protected linear DNA products.

The method may further comprise, after the step of incubating the single contiguous aqueous volume, or the step of contacting the digested DNA with a ligase and the adaptor molecules, a step of purification of the linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. The step of nuclease digestion may allow for removal of any double-stranded DNA molecules and/or adaptor molecules which have not been used to produce linear DNA products. Thus, the method for producing a linear DNA product may comprise the steps:

(a) contacting a double-stranded DNA molecule comprising at least one PAM sequence with an RNP complex comprising at least one gRNA and a cas nuclease, a ligase and first and second adaptor molecules to form a single contiguous aqueous volume;

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and

(c) incubating the single contiguous aqueous volume with a nuclease (e.g. an exonuclease).

Preferably, the cas nuclease is cpf1. The step of contacting the DNA with the RNP complex may be carried out in the presence of $Mg^{2+}$. The double stranded DNA may comprise a PAM sequence 5' of the target sequence.

The method may comprise:

(a) contacting a double-stranded DNA molecule comprising at least one PAM sequence and at least one target sequence with an RNP complex comprising at least one guide RNA (gRNA) and a cas nuclease to form a digested DNA, wherein the at least one gRNA has a sequence complementary to the at least one target sequence;

(b) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region; and

(c) incubating the linear DNA product with a nuclease (e.g. an exonuclease).

Preferably, the cas nuclease is cpf1. The step of contacting the DNA with the RNP complex may be carried out in the presence of $Mg^{2+}$. The double stranded DNA may comprise a PAM sequence 5' of the target sequence.

The method for producing a linear DNA product may comprise the steps:

(a) amplification of a DNA template molecule comprising at least one target sequence and at least one PAM sequence to generate a double-stranded DNA molecule;

(b) contacting the double-stranded DNA molecule with an RNP complex comprising at least one gRNA and a cas nuclease, a ligase and first and second adaptor molecules to form a single contiguous aqueous volume, wherein the at least one gRNA has a sequence complementary to the at least one target sequence;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and

(d) incubating the single contiguous aqueous volume with a nuclease (e.g. an exonuclease).

The method for producing a linear DNA product may comprise the steps:

(a) amplification of a DNA template molecule comprising at least one target sequence and at least one PAM sequence to generate a double-stranded DNA molecule;

(b) contacting a double-stranded DNA molecule with an RNP complex comprising at least one gRNA and a cas nuclease to form a digested DNA, wherein the at least one gRNA has a sequence complementary to the at least one target sequence;

(b) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region; and

(c) incubating the linear DNA product with a nuclease (e.g. an exonuclease).

The method for producing a linear DNA product may comprise the steps:

(a) amplification of a DNA template molecule comprising at least one target sequence and at least one PAM sequence to generate a double-stranded DNA molecule;

(b) contacting the double-stranded DNA molecule with an RNP complex comprising at least one gRNA and a cas nuclease, a ligase and first and second adaptor molecules to form a single contiguous aqueous volume, wherein the at least one guide RNA has a sequence complementary to the at least one target sequence;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region;

(d) purification of the linear DNA product; and

(e) incubating the product of step (d) with a nuclease (e.g. an exonuclease).

The method for producing a linear DNA product may comprise the steps:

(a) amplification of a DNA template molecule comprising at least one target sequence and at least one PAM sequence to generate a double-stranded DNA molecule;

(b) contacting the double-stranded DNA molecule with an RNP complex comprising at least one gRNA and a cas nuclease, a ligase and first and second adaptor molecules to form a single contiguous aqueous volume, wherein the at least one gRNA has a sequence complementary to the at least one target sequence;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region;

(d) incubating the single contiguous aqueous volume with a nuclease (e.g. an exonuclease); and

(e) purification of the linear DNA product.

The method for producing a linear DNA product may comprise the steps:

(a) amplification of a DNA template molecule comprising at least one target sequence and at least one PAM sequence to generate a double-stranded DNA molecule;

(b) contacting the double-stranded DNA molecule with an RNP complex comprising at least one gRNA and a cas nuclease, a ligase and first and second adaptor molecules to form a single contiguous aqueous volume, wherein the at least one gRNA has a sequence complementary to the at least one target sequence;

(c) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region;

(d) incubating the single contiguous aqueous volume with a nuclease (e.g. an exonuclease); and

(e) purification of the linear DNA product.

Preferably, the cas nuclease is cpf1. The step of contacting the DNA with the RNP complex may be carried out in the presence of $Mg^{2+}$. The double stranded DNA may comprise a PAM sequence 5' of the target sequence.

Cpf1 cleaves DNA to leave a 4 or 5 nucleotide 5' overhang. Alternatively, a cas nuclease that creates a blunt end may be used, such as cas9.

The use of a cas nuclease means that the DNA molecule may be cleaved at any point, and a unique sequence can be guaranteed, because the guide RNA can be designed to be complementary to a particular sequence in or outside of the cassette, or sequence of interest. As an alternative to using a restriction enzyme, which may have off-target digestion, the use of a cas nuclease and a guide RNA mitigates the chance of unwanted digestion within the sequence of interest.

The present inventors have surprisingly discovered methods for production of a linear DNA product of enhanced resistance to nuclease digestion. Specifically, the linear DNA product produced by the methods described herein has enhanced resistance to exonuclease digestion (e.g. exonuclease III digestion). The enhanced resistance to exonuclease digestion extends the life of the linear DNA product in a cell (i.e. the linear DNA product has enhanced resistance to intracellular exonucleases) and in a cell-free system (i.e. the linear DNA product has enhanced resistance to extracellular exonucleases). The present inventors have developed a method which relies on the addition of adaptor molecules (i.e. a first adaptor molecule and a second adaptor molecule) to both ends of a double-stranded DNA molecule that has been digested to provide ends to which the adaptors can be ligated. However, unlike previously described methods using protelomerase or restriction endonucleases, the present invention uses a cas nuclease and guide RNA for more precise digestion of DNA. The adaptor molecules may comprise a hairpin or a loop to form a closed linear DNA product with enhanced resistance to nuclease (e.g. exonuclease) digestion. In addition, the present inventors have discovered a method for efficient introduction of protected nucleotides in the form of a first adaptor molecule and a second adaptor molecule at both ends of a linear double-stranded region of the linear DNA product. The methods described herein may use a hairpin or a loop adaptor on one end of the DNA product and a linear adaptor comprising protected nucleotides on the other end of the DNA product. That is to say that any type of adaptors described herein may be used as long as the final DNA product is protected from nuclease (e.g. exonuclease) digestion. The methods of the invention provide protection from digestion by exonucleases that cleave the 3'-end nucleotides (e.g. exonuclease III) and exonucleases that cleave the 5'-end nucleotides (e.g. exonuclease VIII). Thus, the linear DNA product produced by the methods of the invention has prolonged in vivo expression when compared to a linear DNA product that does not comprise an adaptor molecule described herein.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease III digestion). For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The linear DNA product (e.g. the closed linear DNA product) produced by the methods of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear DNA product produced by the methods of the present invention particularly suitable for use in a pharmaceutical composition.

Unexpectedly, the present inventors have discovered a method for production of a linear DNA product of enhanced resistance to nuclease digestion which allows for efficient production of large quantities of the linear DNA product with enhanced resistance to exonuclease digestion. The large-scale manufacture of the product may be in a cell-free system, which results in the production of a pure sample comprising the linear DNA product substantively free of bacterial contaminants (e.g. remaining after cell lysis).

The methods described herein may be used to produce a closed linear DNA product e.g. a covalently closed linear DNA product.

Thus, the invention provides a method for producing a closed linear DNA product, wherein the method comprises:

(a) contacting a double-stranded DNA molecule comprising at least one PAM sequence with an RNP complex comprising at least one gRNA and a cas nuclease, a ligase and first and second adaptor molecules to form a single contiguous aqueous volume; and
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The linear double-stranded region may be the linear portion of the double-stranded DNA molecule.

A closed linear DNA product has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA products offer improved safety. In addition, the closed linear structure reduces concatamerisation of DNA products inside host cells and thus expression levels of the gene product can be regulated in a more sensitive manner.

The methods described herein may be used to produce a linear DNA product comprising nuclease-resistant (i.e. protected nucleotides).

Thus, the method for producing a linear DNA product may comprise the steps:

(a) contacting a double-stranded DNA molecule comprising at least one PAM sequence and at least one target sequence with an RNP complex comprising at least one gRNA and a cas nuclease, a ligase and first and second adaptor molecules to form a single contiguous aqueous volume; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region, and wherein the first and second adaptor molecules are nucleic acid molecules that comprise one or more nuclease-resistant nucleotides (i.e. protected nucleotides).

The linear DNA product produced by the methods described herein has enhanced resistance to nuclease (e.g. exonuclease) digestion. For example, the linear DNA product has prolonged in vivo expression when compared to a linear DNA product that does not contain protected nucleotides

The method of the invention may be used for production of DNA for in vitro expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, $\alpha$1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The closing of the linear double-stranded region by the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The closing of the linear double-stranded region by the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region.

The step of incubating the single contiguous aqueous volume to generate the closed linear DNA product may comprise generating the linear portion of the double-stranded DNA molecule by digesting the double-stranded DNA molecule with the endonuclease.

The step of incubating the single contiguous aqueous volume may be performed under conditions that promote appending (or linking) of the first and second adaptor molecules to the linear double-stranded region to produce the closed linear DNA product. The appending may be performed by creating a covalent link between the first and/or second adaptor molecule and the first and/or second end of the linear double-stranded region.

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C.

The step of incubating the single contiguous aqueous volume may be performed under conditions that promote ligation of the linear double-stranded region to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation:

$$\text{(starting amplified DNA amount) / (final linear DNA amount)} \times 100\%.$$

Ligation efficiency may also be established based on DNA quantification values before and after the digestion/ligation reaction and the subsequent exonuclease treatment to remove remaining open DNA constructs and adaptor molecules excess.

For example, the double-stranded DNA molecule generated by the rolling circle amplification is first quantified so that the amount of the double-stranded DNA molecule used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of cleaving or digesting the DNA with a cas nuclease may be carried out at a temperature of 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The step of ligation of the linear double-stranded region to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the cas nuclease may be cpf1 and the ligase may be T4 DNA ligase, T7 DNA ligase, mammalian DNA ligase I, III and IV; Taq DNA ligase, Tth DNA ligase, or *E. coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C-33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the step of amplifying is performed by rolling circle amplification.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase may not be used. A primer may be used together with a primase. The double-stranded DNA product may be generated by rolling circle amplification in vitro under isothermal conditions using a suitable nucleic acid polymerase, such as Phi29 DNA polymerase.

In the methods described herein, the DNA template molecule may comprise at least one target sequence and at least one PAM sequence, which may be 5' of the target sequence. The at least one target sequence is complementary to the at least one gRNA sequence in the RNP complex. Thus, the DNA template molecule may comprise at least one target sequence complementary to at least gRNA of the RNP complex. Preferably, the DNA template molecule comprises at least two target sequences. Preferably one target sequence is upstream of the sequence of interest or the cassette and one target sequence is downstream of the gene of interest or the cassette. The target sequences may be the same or different. Preferably, the at least one target sequence is 10 to 30 nucleotides in length, preferably 15 to 25 nucleotides in length. The at least one target sequence may be a native sequence (i.e. a sequence present in the template molecule). Alternatively, the at least one target sequence may be introduced to the DNA template molecule prior to the production of the closed linear DNA product.

In the methods described herein, the DNA template molecule may comprise at least one PAM sequence 5' of the target sequence. A PAM sequence is a protospacer adjacent motif and is usually, 2, 3, 4, 5 or 6 base pairs in length. The PAM may be 4 base pairs in length. The PAM sequence may be 3, 4, 5, or 6 base pairs 5' of target sequence to be cleaved by the cas nuclease. If the cas nuclease is cpf1, the PAM sequence is TTTN, wherein N is A, C or G. If the cas nuclease is cas9 the PAM sequence may be NGG.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The template DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The method may further comprise (after the step of amplification and before the step of contacting the double-stranded DNA molecule with an RNP complex comprising at least one gRNA and a cas nuclease, a ligase and first and second adaptor molecules) a step of forming the RNP complex. Thus, the invention provides a method for producing a closed linear DNA product, the method comprises:

(a) amplification of a DNA template molecule comprising at least one target sequence and at least one PAM sequence to generate a double-stranded DNA molecule;
(b) forming an RNP complex comprising a cas nuclease and at least one gRNA, wherein the at least one gRNA has a sequence complementary to the at least one target sequence;
(c) contacting the double-stranded DNA molecule with the RNP complex, a ligase and first and second adaptor molecules to form a single contiguous aqueous volume; and
(d) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The invention provides a method for producing a closed linear DNA product, the method comprises:

(a) amplification of a DNA template molecule comprising at least one target sequence and at least one PAM sequence to generate a double-stranded DNA molecule;

(b) forming an RNP complex comprising a cas nuclease and at least one gRNA, wherein the at least one gRNA has a sequence complementary to the at least one target sequence;

(c) contacting the double-stranded DNA molecule with the RNP complex to generate a digested DNA molecule; and

(d) contacting the digested DNA molecule with a ligase and first and second adaptor molecules to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

Preferably, the RNP complex may be formed by incubating the cas nuclease and the at least one gRNA optionally with a buffer and/or water.

The invention also provides a method for producing a linear DNA product, wherein the method comprises:

(a) contacting a double stranded DNA molecule comprising at least one target sequence and at least one PAM sequence with a composition comprising a cas nuclease and a nucleic acid component, wherein the cas nuclease forms a complex with the nucleic acid component and upon binding of the complex to the at least one target sequence, the cas nuclease makes a cut at the target sequence to produce at least one overhang; and

(b) contacting the at least one overhang with a ligase and at least one adaptor molecule to generate the linear DNA product.

The double stranded DNA molecule may comprise a cassette and a target sequence at each end of the cassette, outside of the cassette, such that an overhang is generated at each end of the cassette.

The step of incubating the single contiguous aqueous volume (or the purified product of step (d)) with a nuclease may be performed at a temperature of 5-90°C, 10-80°C, 15-70°C, 20-60°C, 25-50°C, 30-45°C or 35-40°C. The step of incubating the single contiguous aqueous volume (or the purified product) with a nuclease may be performed for at least 10, at least 20, at least 30, at least 40, at least 50, or at least 60 min. The step of incubating the single contiguous aqueous volume (or the purified product) may be performed at two different temperatures. For example, the step of incubating the single contiguous aqueous volume (or the purified product) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product of step (d)) may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may be a cell-free method.

The closed linear DNA product may be partially double-stranded and/or partially single-stranded. The closed linear DNA product may comprise a portion that is double-stranded and a portion that is single-stranded.

The closed linear DNA product may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The closed linear DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The closed linear DNA product may comprise an inverted terminal repeat sequence.

The closed linear DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the closed linear DNA product is at least 50 base pairs long.

The double-stranded DNA molecule may be circular, or branched.

The double-stranded DNA molecule may not comprise an adaptor. The double-stranded DNA molecule may not comprise a hairpin, a loop or a stem-loop structure.

The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may be a product of amplification. Preferably, the amplification is rolling circle amplification.

The linear double-stranded region (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The linear double-stranded region (e.g. the linear portion of the double-stranded molecule) may comprise a sequence that is at least 40%, 45%, 50%, 55%, 60%, 65%, 70%, 75%, 80%, 85%, 90%, 95%, or 100% identical to the sequence of the double-stranded DNA molecule.

The first end and the second end of the linear double-stranded region (e.g. the linear portion of the double-stranded molecule) may be resistant to nuclease digestion. Preferably, the first end and the second end of the linear double-stranded region are resistant to the exonuclease digestion, such as exonuclease III digestion and/or exonuclease I digestion.

The linear double-stranded region may comprise a 3'-OH group at first and/or second ends. The 3'-OH group may facilitate ligation to the first and/or second adaptor molecule(s) (which may comprise a 5' phosphate). The linear double-stranded region may comprise a 5' phosphate at first and/or second ends. The 5' phosphate may facilitate ligation to the first and/or second adaptor molecule(s) (which may comprise a 3'-OH group).

The linear double-stranded region (e.g. the linear portion of the double-stranded molecule) may comprise an overhang. For example, the linear double-stranded region may comprise a 5' overhang. The linear double-stranded region may comprise a blunt end or blunt ends.

The linear portion of the double-stranded DNA molecule (e.g. the linear portion of the double-stranded molecule) may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, double-stranded DNA molecule is at least 50 base pairs long.

The first adaptor molecule and/or the second adaptor molecule may be a synthetic adaptor molecule.

The first adaptor molecule may be a nucleic acid adaptor molecule. The second adaptor molecule may be a nucleic acid adaptor molecule. The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the first adaptor molecule (e.g. the overhang) may be complementary to the first end of the linear double-stranded region. A portion of the second adaptor molecule may be complementary to the second end of the linear double-stranded region. If the cas nuclease is cpf1, the overhang may be 4 or 5 nucleotides.

The closed linear DNA product may be a covalently closed linear DNA product. Thus, in embodiments where the adaptor molecules comprise a loop (e.g. a hairpin), the adaptor molecules close the ends of the linear double-stranded region forming a covalently closed linear DNA product.

The first adaptor molecule and/or the second adaptor molecule may not be a plasmid or a vector DNA.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first adaptor molecule and/or the second adaptor molecule may comprise the sequence of SEQ ID NO: 4 to 15 or a portion thereof. The first adaptor molecule and/or the second adaptor molecule may comprise at least 13, at least 14, at least 15, at least 16, at least 17, at least 18, or at least 19 contiguous nucleotides of SEQ ID NO: 4 to 15. The double-stranded portion of the first adaptor molecule and/or the second adaptor molecule may comprise the sequence of SEQ ID NO: 4 to 15 or a portion thereof. The double-stranded portion of the first adaptor molecule and/or the second adaptor molecule may comprise at least 8, at least 9, at least 10, at least 11, at least 12, at least 13, at least 14 or at least 15 contiguous nucleotides of SEQ ID NO: 4 to 15. The single-stranded portion of the first adaptor molecule and/or the second adaptor molecule may comprise a sequence of ACTCA. The single-stranded portion of the first adaptor molecule and/or the second adaptor molecule may comprise at least 1, at least 2, at least 3, at least 4 or at least 5 contiguous nucleotides of the sequence ACTCA. The first and second adapter molecules may comprise an identical nucleic acid sequence. The first and second adapter molecules may comprise a different nucleic acid sequence.

The first adaptor molecule may comprise a portion that is complementary to the first end of the linear double-stranded region (or the linear portion of the double-stranded DNA molecule). The second adaptor molecule may comprise a portion that is complementary to the second end of the linear double-stranded region (or the linear portion of the double-stranded DNA molecule). The first adaptor molecule may comprise a portion that anneals to the first end of the linear double-stranded region (or the linear portion of the double-stranded DNA molecule). The second adaptor molecule may comprise a portion that anneals to the second end of the linear double-stranded region (or the linear portion of the double-stranded DNA molecule). The first adaptor molecule may comprise a portion that is complementary and anneals to the first end of the linear double-stranded region (or the linear portion of the double-stranded DNA molecule). The second adaptor molecule may comprise a portion that is complementary and anneals to the second end of the linear double-stranded region (or the linear portion of the double-stranded DNA molecule).

The portion that is complementary or anneals to the first or second end of the linear double-stranded region (or the linear portion of the double-stranded DNA molecule) may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The nucleotides resistant to exonuclease digestion (i.e. protected nucleotides) suitable for use in the methods described herein may be phosphorothioated nucleotides. For example, phosphorothioated nucleotides may be $\alpha$-S-dATP (i.e. 2'-deoxyadenosine-5'-($\alpha$-thio)-triphosphate), $\alpha$-S-dCTP (i.e. 2'-deoxycytidine-5'-($\alpha$-thio)-triphosphate), $\alpha$-S-dGTP (i.e. 2'-deoxyguanosine-5'-($\alpha$-thio)-triphosphate), $\alpha$-S-dTTP (i.e. 2'-deoxythymidine-5'-($\alpha$-thio)-triphosphate), $\alpha$-S-dUTP (i.e. 2'-deoxyuridine-5'-($\alpha$-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the closed linear DNA product. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the DNA product, the functional portion may comprise a fluorophore, a radioactive compound or a barcode.

A signal corresponding to the presence, absence and/or level of the closed linear DNA product may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the closed linear DNA product. The binding moiety is capable of binding to 3' and/or 5' end of the closed linear DNA product. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the closed linear DNA product and the second binding moiety linked to the second barcoded portion may bind to the 5' end of the closed linear DNA product. The 3' and 5' ends may comprise a 3' overhang, a 5' overhang or a blunt end.

A signal corresponding to the presence, absence and/or level of the closed linear DNA product may be measured using a fluorophore (i.e. a fluorescently-labelled molecule), which is attached or bound to the 3' overhang, the 5' overhang or the blunt end of the closed linear DNA product. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeq™, MiSeq™ and/or Genome Analyzer™ sequencing systems), Oxford Nanopore™ Technologies (e.g. the MinION sequencing system), Ion Torrent™ (e.g. the Ion PGM™ and/or Ion Proton™ sequencing systems), Pacific Biosciences (e.g. the PACBIO RS II sequencing system); Life Technologies™ (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The first adaptor molecule and/or the second adaptor molecule may confer resistance to the nuclease digestion, such as exonuclease digestion (e.g. exonuclease I and/or exonuclease III digestion).

The closing of the linear double-stranded region (or the linear portion of the double-stranded DNA molecule) at the first end may generate a first closed end of the closed linear DNA product. The closing of the linear double-stranded region (or the linear portion of the double-stranded DNA molecule) at the second end may generate a second closed end of the closed linear DNA product. The first closed end and the second closed end of the closed linear DNA product may be resistant to nuclease digestion. The nuclease digestion may be exonuclease digestion. Preferably, the nuclease digestion is exonuclease III digestion and/or exonuclease I digestion.

The first and second adaptor molecules may comprise one or more phosphorothioated nucleotides, such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the linear double-stranded region, the linear DNA product may comprise a protected nucleotide (e.g. phosphorothioated nucleotide) at the 5'-end (or at the 5'-end region) of one or both strands. Preferably, the linear DNA product comprises a phosphorothioated nucleotide at the 5'-end (or at the 5'-end region) of one or both strands. The linear DNA product may comprise a phosphorothioated nucleotide at the 5'-end (or at the 5'-end region) of one or both strands. As most exonucleases, for example exonuclease III, remove nucleotides from the 3'-end of the polynucleotide chain, the linear DNA product may comprise a protected nucleotide at the 3'-end (or at the 3'-end region) of one or both strands. Preferably, the linear DNA product comprises a phosphorothioated nucleotide at the 3'-end (or the 3'-end region) of one or both strands. The linear DNA product may comprise at least one phosphorothioated nucleotide at the 3'-end (or the 3'-end region) and at least one phosphorothioated nucleotide at the 5'-end (or the 5'-end region) of one or both strands. The linear DNA product may comprise a phosphorothioated nucleotide at the 3'-end (or the 3'-end region) and the 5'-end (or the 5'-end region) of one or both strands.

The nucleotides resistant to exonuclease digestion (i.e. protected nucleotides) may be phosphorothioated nucleotides of at least one type. For example, the at least one type of phosphorothioated nucleotides is $\alpha$-S-dATP (i.e. 2'-deoxyadenosine-5'-($\alpha$-thio)-triphosphate), $\alpha$-S-dCTP (i.e. 2'-deoxycytidine-5'-($\alpha$-thio)-triphosphate), $\alpha$-S-dGTP (i.e. 2'-deoxyguanosine-5'-($\alpha$-thio)-triphosphate), $\alpha$-S-dTTP (i.e. 2'-deoxythymidine-5'-($\alpha$-thio)-triphosphate), $\alpha$-S-dUTP (i.e. 2'-deoxyuridine-5'-($\alpha$-thio)-triphosphate), and/or uridine 2', 3'-cyclophosphorothioate.

The adaptor molecules may comprise at least two types of phosphorothioated nucleotides. For example, the at least two types of phosphorothioated nucleotides are: $\alpha$-S-dATP and $\alpha$-S-dCTP, $\alpha$-S-dATP and $\alpha$-S-dGTP, $\alpha$-S-dATP and $\alpha$-S-dTTP, $\alpha$-S-dCTP and $\alpha$-S-dGTP, $\alpha$-S-dCTP and $\alpha$-S-dTTP, or $\alpha$-S-dGTP and $\alpha$-S-dTTP.

The adaptor molecules may comprise at least three types of phosphorothioated nucleotides. For example, the at least three types of phosphorothioated nucleotides are:

(a) $\alpha$-S-dATP, $\alpha$-S-dCTP and $\alpha$-S-dGTP;
(b) $\alpha$-S-dATP, $\alpha$-S-dCTP and $\alpha$-S-dTTP;
(c) $\alpha$-S-dATP, $\alpha$-S-dGTP and $\alpha$-S-dTTP; or
(d) $\alpha$-S-dCTP, $\alpha$-S-dGTP and $\alpha$-S-dTTP.

The adaptor molecules may comprise at least four types of phosphorothioated nucleotides. For example, the at least four types of protected nucleotides are $\alpha$-S-dATP, $\alpha$-S-dCTP, $\alpha$-S-dGTP and $\alpha$-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The adaptor molecules are ligated at both sides of the expression cassette, resulting in a linear protected DNA product due to the presence of protected nucleotides (marked with asterisk) at both sides and both strands of the expression cassette, which prevents exonuclease degradation of the linear DNA product.

The methods described herein may be used to produce a partially closed linear DNA product comprising nuclease-resistant (i.e. protected nucleotides).

Thus, the method for producing a partially closed linear DNA product may comprise the steps:

(a) contacting a double-stranded DNA molecule comprising at least one target sequence and at least one PAM sequence with an RNP complex comprising at least one gRNA and a cas nuclease, a ligase and first and second adaptor molecules to form a single contiguous aqueous volume, wherein the at least one gRNA has a sequence complementary to the at least one target sequence; and
(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region, and wherein the first adaptor molecule is a nucleic acid molecule that comprises one or more nuclease-resistant nucleotides (i.e. protected nucleotides) and wherein the linear double-stranded region is closed at the second end by the second adaptor molecule.

The method for producing a partially closed linear DNA product may comprise the steps:

(a) contacting a double-stranded DNA molecule comprising at least one target sequence and at least one PAM sequence with an RNP complex comprising at least one gRNA and a cas nuclease to form a digested DNA molecule, wherein the at least one gRNA has a sequence complementary to the at least one target sequence; and
(b) contacting the digested DNA molecule with a ligase and first and second adaptor molecules to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region, and wherein the first adaptor molecule is a nucleic acid molecule that comprises one or more nuclease-resistant nucleotides (i.e. protected nucleotides) and wherein the linear double-stranded region is closed at the second end by the second adaptor molecule.

The partially closed linear DNA product produced by the methods described herein has enhanced resistance to nuclease (e.g. exonuclease) digestion.

Self-complementary adaptor molecules may be ligated at the upstream side of the expression cassette. Downstream adaptors may be formed by hybridization of complementary oligonucleotides containing phosphorothioate internucleotide linkages (marked as asterisks) and forming a 4-nucleotide protruding end at 5'. Complementary adaptor molecules are ligated at each side of the expression cassette, resulting in partially closed linear DNA product which has enhanced resistance to exonuclease, since phosphorothioate internucleotide linkages at one sides of the expression cassette prevent exonucleolytic degradation of the partially closed linear DNA product.

**Methods for transcription and protein expression**

The invention provides a method for in vitro transcription of a linear DNA product (e.g. a closed linear DNA product or a partially closed linear DNA product), wherein the method comprises contacting the linear DNA product (e.g. a closed linear DNA product or a partially closed linear DNA product), produced by the methods described herein, with a polymerase and producing a transcription product encoded by the linear DNA product (e.g. the closed linear DNA product or a partially closed linear DNA product).

The invention provides a method for in vitro transcription of a linear DNA product (e.g. a closed linear DNA product or a partially closed linear DNA product), wherein the method comprises:

(a) producing a linear DNA product (e.g. a closed linear DNA product or a partially closed linear DNA product) by any of the methods described herein;
(b) contacting the linear DNA product (e.g. the closed linear DNA product or a partially closed linear DNA product), with a polymerase; and
(c) producing a transcription product encoded by the linear DNA product (e.g. the closed linear DNA product or a partially closed linear DNA product).

The invention provides a method for in vitro transcription of a linear DNA product, wherein the method comprises:

(a) contacting a double-stranded DNA molecule comprising at least one target sequence and at least one PAM sequence with an RNP complex comprising a cas nuclease and at least one guide RNA (gRNA), and a ligase and first and second adaptor molecules to form a single contiguous aqueous volume, wherein the at least one gRNA has a sequence complementary to the at least one target sequence; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and
(c) contacting the linear DNA product with a polymerase; and
(d) producing a transcription product encoded by the linear DNA product.

The invention provides a method for in vitro transcription of a linear DNA product, wherein the method comprises:

(a) contacting a double-stranded DNA molecule comprising at least one target sequence and at least one PAM sequence with an RNP complex comprising a cas nuclease and at least one guide RNA (gRNA) to generate a digested DNA molecule, wherein the at least one gRNA has a sequence complementary to the at least one target sequence;
(b) contacting the digested DNA molecule with a ligase and first and second adaptor molecules to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and
(c) contacting the linear DNA product with a polymerase; and
(d) producing a transcription product encoded by the linear DNA product.

The method may use adaptor molecules which generate a closed linear DNA product, such as adaptor molecules described herein. A method for in vitro transcription of a closed linear DNA product may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;
(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule;
(c) contacting the closed linear DNA product with a polymerase; and
(d) producing a transcription product encoded by the closed linear DNA product.

The method may use adaptor molecules which comprise protected nucleotides, such as the adaptor molecules described herein. A method for in vitro transcription of a linear DNA product may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides (i.e. protected nucleotides);
(c) contacting the linear DNA product with a polymerase; and
(d) producing a transcription product encoded by the linear DNA product.

The method may use adaptor molecules which comprise protected nucleotides and adaptor molecules which comprise a hairpin or a stem loop, such as the adaptor molecules described herein. A method for in vitro transcription of a partially closed linear DNA product may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;
(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule;
(c) contacting the partially closed linear DNA product with a polymerase; and
(d) producing a transcription product encoded by the partially closed linear DNA product.

The invention provides a method for producing a protein, wherein the method comprises introducing the linear DNA product (e.g. the closed linear DNA product or the partially closed linear DNA product), produced by the methods described herein, into a cell (e.g. a prokaryotic cell or a eukaryotic cell) or a cell-free expression system to generate a protein encoded by the linear DNA product (e.g. the closed linear DNA product or the partially closed linear DNA product).
The invention provides a method for producing a protein, wherein the method comprises:

(a) producing a linear DNA product (e.g. a closed linear DNA product or partially closed linear DNA product) by any of the methods described herein; and
(b) introducing the linear DNA product (e.g. the closed linear DNA product or partially closed linear DNA product) into a cell (e.g. a prokaryotic cell or a eukaryotic cell) or a cell-free expression system to generate a protein encoded by the linear DNA product (e.g. the closed linear DNA product or partially closed linear DNA product).

The invention provides a method for producing a protein comprising:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and

(c) introducing the linear DNA product into a cell (e.g. a prokaryotic cell or a eukaryotic cell) or a cell-free expression system to generate a protein encoded by the linear DNA product.

The invention provides a method for producing a protein comprising:

(a) contacting a double-stranded DNA molecule comprising at least one target sequence and at least one PAM sequence with an RNP complex comprising a cas nuclease and at least one guide RNA (gRNA) to generate a digested DNA molecule;

(b) contacting the digested DNA molecule with a ligase and first and second adaptor molecules to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and

(c) introducing the linear DNA product into a cell (e.g. a prokaryotic cell or a eukaryotic cell) or a cell-free expression system to generate a protein encoded by the linear DNA product.

The method may use adaptor molecules which generate a closed linear DNA product, such as adaptor molecules described herein. A method for producing a protein may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;

(b) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule; and

(c) introducing the closed linear DNA product into a cell (e.g. a prokaryotic cell or a eukaryotic cell) or a cell-free expression system to generate a protein encoded by the closed linear DNA product.

The method may use adaptor molecules which comprise protected nucleotides, such as the adaptor molecules described herein. A method for producing a protein may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides (i.e. protected nucleotides); and

(c) introducing the linear DNA product into a cell (e.g. a prokaryotic cell or a eukaryotic cell) or a cell-free expression system to generate a protein encoded by the linear DNA product.

The method may use adaptor molecules which generate a partially closed linear DNA product, such as adaptor molecules described herein. A method for producing a protein may comprise:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume;

(b) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule; and

(c) introducing the partially closed linear DNA product into a cell (e.g. a prokaryotic cell or a eukaryotic cell) or a cell-free expression system to generate a protein encoded by the partially closed linear DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The linear DNA product or the closed linear DNA product may comprise a cassette. The desired protein might be encoded by the cassette.

The step of introducing the linear DNA product into a cell may be performed in vivo or in vitro.

The nuclease-resistant nucleotides (i.e. protected nucleotides) may be any protected nucleotides described herein.

## Methods for cell transfection and cell transfection compositions

The invention provides a method for cell transfection of a linear DNA product (e.g. a closed linear DNA product or a partially closed linear DNA product), produced by any of the methods described herein, into a cell.

The invention provides a method for cell transfection of a linear DNA product (e.g. a closed linear DNA product or a partially closed linear DNA product) into a cell, wherein the method comprises:

(a) producing a linear DNA product (e.g. a closed linear DNA product or a partially closed linear DNA product) by any of the methods described herein;
(b) contacting a cell with the linear DNA product (e.g. the closed linear DNA product or a partially closed linear DNA product); and
(c) transfecting the linear DNA product (e.g. the closed linear DNA product or a partially closed linear DNA product) into the cytosol of the cell.

The invention provides a method for cell transfection of a linear DNA into a cell, wherein the method comprises:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region;
(c) contacting the linear DNA product with the cell; and
(d) transfecting the linear DNA product into the cytosol of the cell.

The invention provides a method for cell transfection of a linear DNA into a cell, wherein the method comprises:

(a) contacting a double-stranded DNA molecule comprising at least one target sequence and at least one PAM sequence with an RNP complex comprising a cas nuclease and at least one guide RNA (gRNA) to generate a digested DNA molecule;
(b) contacting the digested DNA molecule with a ligase and first and second adaptor molecules to generate the linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region;

(c) contacting the linear DNA product with the cell; and

(d) transfecting the linear DNA product into the cytosol of the cell.

The method may use adaptor molecules which generate a closed linear DNA product, such as adaptor molecules described herein.

The method may use adaptor molecules which comprise protected nucleotides and adaptor molecules comprising a hairpin, such as the adaptor molecules described herein.

The method may use adaptor molecules which comprise protected nucleotides, such as the adaptor molecules described herein.

The invention provides a cell transfection composition comprising a linear DNA product (e.g. a closed linear DNA product) produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

The invention further provides a cell transfection method comprising contacting (in vitro) a cell to be transfected with a linear DNA product (e.g. a closed linear DNA product) produced by the methods of the invention, and wherein the linear DNA product (e.g. the closed linear DNA product) is transfected into the cytosol of the cell.

The cell or cells to be transfected may be provided in a cell culture medium (e.g. in a Petri dish, culture vessel or well, etc). The linear DNA product (e.g. the closed linear DNA product) may be added directly to the cell culture medium or the cells may be added to a solution, such as saline, a buffered solution or a cell culture medium, comprising the linear DNA product (e.g. the closed linear DNA product).

The carrier may be a viral carrier or a non-viral carrier. Viral carriers include a lentiviral vector, an adenoviral vector, a retroviral vector, or an adeno associate viral vector for delivery of the linear DNA product. Non-viral carriers (or vectors) include complexing the linear DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product (e.g. the closed linear DNA product).

The carrier may be a modification of the linear DNA product with a targeting ligand (e.g. an antibody), a peptide, a small molecule (e.g. folic acid and/or biotin), or a polymer present in extracellular matrix

(e.g. hyaluronic acid and/or chondroitin sulphate), or a hydrophobic modification of the double-stranded nucleic acid molecule (e.g. using cholesterol and/or $\alpha$-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

The invention further provides a cell obtainable by the methods of the invention. Thus, the cell may comprise a linear DNA product (e.g. the closed linear DNA product) produced by the methods of the invention.

The invention further provides a cell transfected with a linear DNA product (e.g. the closed linear DNA product) produced by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear DNA product (e.g. the closed linear DNA product) may be performed in vivo. For example the linear DNA product (e.g. the closed linear DNA product) may be administered to an organism (e.g. a subject) in need thereof. The organism (e.g. the subject) may be an animal, such as mammal (e.g. a human), a fungus, a micro-organism, or a plant.

Any or all of the linear DNA products (e.g. the closed linear DNA products) described herein may be delivered to a cell via delivery particles such as liposomes, nanoparticles, exosomes, macrovesicles, viral or non-viral vectors. Any or all of the linear DNA products (e.g. the closed linear DNA products) described herein may be delivered to a cell using a gene-gun. Any or all of the linear DNA products (e.g. the closed linear DNA products) described herein may be delivered to a cell by electroporation. Any or all of the linear DNA products (e.g. the closed linear DNA products) described herein may be delivered to a cell by hydrodynamic needle. The linear DNA products (e.g. the closed linear DNA products) described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The DNA product and the lipid component may reversibly interact to form a self-assembled nanoparticle. The DNA product and the lipid component may reversibly interact in the self-assembled nanoparticle through intermolecular forces. The DNA product and the lipid component may reversibly interact in the self-assembled nanoparticle through non-covalent interactions. The DNA product and the lipid component may reversibly interact in the self-assembled nanoparticle through hydrogen bonds, van der Waals, hydrophobic, and/or electrostatic interactions. The DNA product and the lipid component may not be conjugated or linked by forces other than inter-molecular forces in the self-assembled nanoparticle.

**Pharmaceutical compositions and methods for producing pharmaceutical compositions**

The invention provides a pharmaceutical composition comprising a linear DNA product (e.g. a closed linear DNA product) described herein, and a pharmaceutically acceptable carrier or excipient.

The invention provides a pharmaceutical composition comprising a linear DNA product (e.g. a closed linear DNA product) produced by (or obtainable by) the methods described herein, and a pharmaceutically acceptable carrier or excipient.

The invention provides a method for producing a pharmaceutical composition comprising the linear DNA product, wherein the method comprises performing the method described herein and formulating the resulting linear DNA product with a pharmaceutically acceptable carrier or excipient.

The invention provides a method for producing a pharmaceutical composition, wherein the method comprises:

(a) producing a linear DNA product (e.g. a closed linear DNA product) by any of the methods described herein;
(b) formulating the linear DNA product (e.g. the closed linear DNA product) with a pharmaceutically acceptable carrier or excipient.

The invention provides a method for producing a pharmaceutical composition, wherein the method comprises:

(a) contacting a double-stranded DNA molecule comprising at least one target sequence and at least one PAM sequence with an RNP complex comprising a cas nuclease and at least one guide RNA (gRNA), a ligase and first and second adaptor molecules to form a single contiguous aqueous volume, wherein the at least one gRNA has a sequence complementary to the at least one target sequence; and
(b) incubating the single contiguous aqueous volume to generate a linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and
(c) formulating the linear DNA product with a pharmaceutically acceptable carrier or excipient.

The invention provides a method for producing a pharmaceutical composition, wherein the method comprises:

(a) contacting a double-stranded DNA molecule comprising at least one target sequence and at least one PAM sequence with an RNP complex comprising a cas nuclease and at least one guide RNA (gRNA) to generate a digested DNA molecule, wherein the at least one gRNA has a sequence complementary to the at least one target sequence; and
(b) contacting the digested DNA molecule with a ligase and first and second adaptor molecules to generate a linear DNA product, wherein the linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region; and
(c) formulating the linear DNA product with a pharmaceutically acceptable carrier or excipient

The cas nuclease may be cpf1. The double stranded DNA molecule may comprise a PAM sequence 5' of the target sequence, which may be TTTN.

The method may use adaptor molecules which generate a closed linear DNA product, such as adaptor molecules described herein.

The method may use adaptor molecules which comprise protected nucleotides, such as the adaptor molecules described herein.

The method may use adaptor molecules which generate a partially closed linear DNA product, such as adaptor molecules described herein.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear DNA product (e.g. closed linear DNA product) can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochiear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a mannerthat liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

**Linear DNA products**

The invention provides a linear DNA product (e.g. a closed linear DNA product) as described herein.

The invention provides a closed linear DNA product comprising a linear double-stranded region, wherein the linear double-stranded region comprises a linear portion of a double-stranded DNA molecule, and wherein the linear double-stranded region is closed at a first end by a first adaptor molecule and closed at a second end by the second adaptor molecule.

The invention provides a closed linear DNA product comprising a linear portion of a double-stranded DNA molecule, wherein the linear portion of the double-stranded DNA molecule is closed at a first end by a first adaptor molecule and closed at a second end by a second adaptor molecule.

The invention provides a linear DNA product comprising a linear double-stranded region, wherein the linear double-stranded region comprises a linear portion of a double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention provides a linear DNA product comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first and second adaptor molecules are nucleic acid molecules that comprise one or more nuclease-resistant nucleotides.

The invention provides a partially closed linear DNA product comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprise one or more nuclease-resistant nucleotides, and wherein the linear portion of the double-stranded DNA molecule is closed at the second end by the second adaptor molecule. Thus, the invention provides a partially closed linear DNA product which is closed (or covalently closed) at a second end and open at a first end. The partially closed linear DNA product comprises one or more nuclease-resistant nucleotides in the open-end region and/or at the first end. The open-end region may be at the 3' end or 5' end of the molecule.

The invention provides a linear DNA product (e.g. a closed linear DNA product or a partially closed linear DNA product) obtainable by any of the methods described herein.

**Uses and applications**

The invention provides a use of a linear DNA product (e.g. a closed linear DNA product) as described herein in the production of viral or non-viral delivery system.

The invention provides a use of a linear DNA product (e.g. a closed linear DNA product) in the production of viral or non-viral delivery system, wherein the linear DNA product (e.g. the closed linear DNA product) is produced by performing the method described herein.

The invention provides a viral or non-viral delivery system comprising a linear DNA product (e.g. a closed linear DNA product) as described herein. The invention provides a viral or non-viral delivery system comprising a linear DNA product (e.g. a closed linear DNA product), wherein the linear DNA product (e.g. the closed linear DNA product) is produced by performing the method described herein.

Viral vectors

Methods of producing viral vectors, such as AAV vectors, are known in the art. The most widely used method involves the co-transfection of HEK293 by three bacterial plasmids. The first plasmid encodes the Rep and Cap element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product (e.g. a closed linear DNA product) suitable for use in production of viral vectors. The linear DNA product (e.g. the closed linear DNA product) overcomes the above issues with plasmid vectors.

The invention provides a method of producing a viral vector, the method comprising introducing the linear DNA product (e.g. a closed linear DNA product) produced by the methods described herein into a cell under conditions such that the viral vector is produced. The linear DNA product (e.g. the closed linear DNA product) may encode at least one element required for the production of the viral vector. For example, the linear DNA product (e.g. the closed linear DNA product) may encode Rep and/or Cap elements. The linear DNA product (e.g. the closed linear DNA product) may encode the helper plasmid elements. The linear DNA product (e.g. the closed linear DNA product) may encode Rep, Cap and helper plasmid elements. The linear DNA product (e.g. the closed linear DNA product) may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the closed linear DNA product) to a cell, comprising contacting the viral vector produced by the methods described herein with the cell. The cell may be an animal cell, preferably mammal cell, such as human cell.

The invention also provides a cell obtainable by the methods described herein.

Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product (e.g. a closed linear DNA product) produced by the methods described herein is suitable for use in production of non-viral vectors. The linear DNA product (e.g. the closed linear DNA product) produced by the methods of the invention overcomes the issues of using plasmid vectors in non-viral vector preparation. For example, the linear DNA product (e.g. the closed linear DNA product), unlike plasmid DNA, does not comprise a bacterial backbone, allowing more transgene copies per mg of DNA. In addition, the linear DNA product (e.g. the closed linear DNA product) does not comprise antibiotic resistant genes and bacterial contaminants. Moreover, the linear DNA product (e.g. the closed linear DNA product) provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product (e.g. a closed linear DNA product) with the cell. The cell may be an animal cell, preferably mammal cell, such as human cell.

The invention also provides a cell obtainable by the methods described herein.

General therapeutic and diagnostic uses

The linear DNA product (e.g. a closed linear DNA product) produced by the methods described herein is particularly suitable for use in therapy. The invention provides a linear DNA product (e.g. a closed linear DNA product) as described herein for use in therapy. The invention provides a linear DNA product (e.g. a closed linear DNA product) obtainable by the method described herein for use in therapy. The linear DNA product (e.g. the closed linear DNA product) may encode a sequence of a therapeutic protein, a part of a vaccine, or an element of a genetic engineering mechanism, which is used to treat a disease or infection in a subject.

The invention further provides the linear DNA product (e.g. a closed linear DNA product) as described herein for use as a medicament. The invention further provides the linear DNA product (e.g. a closed linear DNA product) obtainable by the methods described herein for use as a medicament. The invention also provides the use of a linear DNA product (e.g. a closed linear DNA product) as described herein for the manufacture of a medicament for treating a disease. The invention also provides the use of a linear DNA product (e.g. a closed linear DNA product) obtainable by the methods described herein for the manufacture of a medicament for treating a disease.

The linear DNA product (e.g. the closed linear DNA product) may encode a sequence of a therapeutic protein, a part of a vaccine, or an element of a genetic engineering mechanism, which is used to treat a disease or infection in a subject.

The invention further provides the linear DNA product produced by the methods described herein for use in treating a disease.

The invention also provides a method of treating a disease in a subject comprising administering to the subject a linear DNA product (e.g. a closed linear DNA product) described herein. The invention also provides a method of treating a disease in a subject comprising administering to the subject the linear DNA product (e.g. a closed linear DNA product) obtainable by the methods described herein. Preferably, the amount of the linear DNA product administered to the subject is a therapeutic active amount.

The linear DNA product (e.g. the closed linear DNA product) described herein may be used to treat any disease or disorder. For example, the linear DNA product (e.g. the closed linear DNA product) may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product (e.g. the closed linear DNA product) is used to treat a genetic disorder. More preferably still, the linear DNA product (e.g. the closed linear DNA product) is used to treat a monogenic disorder. For example, the linear DNA product (e.g. the closed linear DNA product), may be used to treat sickle cell anaemia, cystic fibrosis, Huntington disease, and Duchenne's Muscular Dystrophy, Haemophilia A, $\alpha$1-antitrypsin deficiency, primary ciliary dyskinesia, or respiratory distress syndrome of prematurity.

A subject treated with the linear DNA product (e.g. a closed linear DNA product) may receive the linear DNA product (e.g. a closed linear DNA product) in the form of any of the pharmaceutical compositions described herein.

A subject treated with the linear DNA product (e.g. a closed linear DNA product) may receive the linear DNA product (e.g. the closed linear DNA product) in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the linear DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the linear DNA product (e.g. a closed linear DNA product) as described herein in a method of diagnosing a disease and/or a disorder. The invention also provides the use of the linear DNA product (e.g. the closed linear DNA product) obtainable by the methods described herein in a method of diagnosing a disease and/or a disorder.

The invention provides the use of the linear DNA product (e.g. the closed linear DNA product) in the "in vitro" diagnosis of a disease. The invention provides the use of the linear DNA product (e.g. the closed linear DNA product) obtainable by the methods described herein in the "in vitro" diagnosis of a disease.

The invention also provides the linear DNA product (e.g. the closed linear DNA product) for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to diagnose a genetic disorder. More preferably still, the linear DNA product is used to diagnose a monogenic disorder. For example, the linear DNA product may be used to diagnose sickle cell anaemia, cystic fibrosis, Huntington disease, and Duchenne's Muscular Dystrophy, Haemophilia A, $\alpha$1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear DNA product (e.g. closed linear DNA product).

To facilitate detection and/or quantification of the linear DNA product, the linear DNA product (e.g. the closed linear DNA product) may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the linear DNA product. For example, the signal may be measured by flow cytometry and/or fluorescence-activated cell sorting of a linear DNA product attached to a fluorescent probe.

The linear DNA product (e.g. the closed linear DNA product) may be detected by being bound to a capture moiety, for example in a lateral flow assay. In this example, the functional portion is a protein, for example, an antibody specific for the capture moiety. The capture of the antibody attached to the linear DNA product may produce a visual signal (e.g. a band of a different colour).

The invention also provides a method that combines diagnosis of a disease with a treatment of the disease.

Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the linear DNA product (e.g. the closed linear DNA product) may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product (e.g. the closed linear DNA product) described herein for use in cell therapy. The invention provides the linear DNA product (e.g. the closed linear DNA product) obtainable by the methods described herein for use in cell therapy.

The invention provides the linear DNA product (e.g. the closed linear DNA product) described herein for use in cell therapy. The invention provides the linear DNA product (e.g. the closed linear DNA product) obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

Vaccines

The linear DNA products (e.g. the closed linear DNA products) produced by the methods described herein are particularly suitable for use in vaccine production. A vaccine may comprise a linear DNA product (e.g. a closed linear DNA product) described herein. A vaccine may comprise a linear DNA product (e.g. a closed linear DNA product) obtainable by the methods described herein. Alternatively, the linear DNA product (e.g. the closed linear DNA product) may be used to produce a vaccine, preferably an mRNA-based vaccine. For example, vaccines such as the BioNTech and Moderna mRNA vaccines against COVID-19.

Thus, the invention provides the use of the linear DNA product (e.g. the closed linear DNA product) described herein in the production of a vaccine. The invention also provides the use of the linear DNA product (e.g. the closed linear DNA product) obtainable by the methods described herein in the production of a vaccine.

The linear DNA product (e.g. the closed linear DNA product) may encode an antigen, which may cause an immune response in a subject. The subject may be human. Preferably, the antigen is encoded on the cassette.

CAR-T cells

The invention provides the use of a linear DNA product (e.g. the closed linear DNA product) described herein in the production of a CAR-T cell. The invention provides the use of the linear DNA product (e.g. the closed linear DNA product) obtainable by the methods described herein in the production of a CAR-T cell.

The invention provides a method for producing a genetically engineered CAR-T cell comprising: (a) introducing the linear DNA product (e.g. a closed linear DNA product) described herein into a T cell; and (b) expressing a gene of interest encoding by the linear DNA product (e.g. the closed linear DNA product). Preferably, the gene of interest is a tumour-specific CAR.

The invention provides a method for producing a genetically engineered CAR-T cell comprising: (a) introducing the linear DNA product (e.g. a closed linear DNA product) obtainable by the methods described herein into a T cell; and (b) expressing a gene of interest encoding by the linear DNA product (e.g. the closed linear DNA product). Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

The invention also provides an engineered T cell obtainable by any methods described herein. The engineered T cell may be suitable for use in CAR T-cell therapy.

CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product (e.g. the closed linear DNA product) may comprise a gene sequence encoding any component of the CRIPSR machinery. The linear DNA product (e.g. the closed linear DNA product) may encode all components of the CRIPSR machinery.

The linear DNA product (e.g. the closed linear DNA product) may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product (e.g. the closed linear DNA product) encoding the repair template may be delivered to a cell by a nanoparticle, a non-viral vector or a viral vector, or without the aid of any carrier. The linear DNA product (e.g. the closed linear DNA product) encoding the repair template may be delivered to a cell by electroporation. The linear DNA product (e.g. the closed linear DNA product) encoding the repair template may be delivered to a cell by hydrodynamic needle.

The linear DNA product (e.g. the closed linear DNA product) may comprise (or further comprise) a gene sequence encoding a nuclease protein of a CRISPR system (e.g. Cas9, Cpf1, or MAD7), and/or a guide RNA. The linear DNA product (e.g. the closed linear DNA product) may comprise (or further comprise) a gene sequence encoding a nuclease protein of a CRISPR system (e.g. Cas9, Cpf1, or MAD7). The linear DNA product (e.g. the closed linear DNA product) may comprise (or further comprise) a gene sequence encoding a guide RNA. The linear DNA product (e.g. the closed linear DNA product) may comprise (or further comprise) a gene sequence encoding a nuclease protein of a CRISPR system (e.g. Cas9, Cpf1, or MAD7) and a guide RNA. The linear DNA product (e.g. the closed linear DNA product) may comprise (or further comprise) a gene sequence encoding a genomic target to be modified (e.g. a spacer). The linear DNA product (e.g. the closed linear DNA product) may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the linear DNA product (e.g. the closed linear DNA product) may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product (e.g. the closed linear DNA product) may encode the nuclease of the CRISPR system and guide RNA. One linear DNA product (e.g. one closed linear DNA product) may encode the nuclease of the CRISPR system, and the other linear DNA product (e.g. the other closed linear DNA product) may encode guide RNA.

The linear DNA product (e.g. the closed linear DNA product) may be for use in CRISPR-Cas mediated repair by recombination, homology-directed repair, or non-homologous end joining.

If the nuclease of the CRISPR system and the guide RNA are encoded by a different linear DNA product (e.g. a different closed linear DNA product), they may be part of a different or the same delivery mechanism. For example, the linear DNA product (e.g. the closed linear DNA product) encoding the nuclease of the CRISPR system may be delivered to a cell by a first nanoparticle, non-viral vector or viral vector, whereas the linear DNA product (e.g. the closed linear DNA product) encoding the guide RNA may be delivered to a cell by a second nanoparticle, a non-viral vector, or viral vector. For example, the linear DNA product (e.g. the closed linear DNA product) encoding the nuclease of the CRISPR system and the linear DNA product (e.g. the closed linear DNA product) encoding the guide RNA may be delivered to a cell by the same nanoparticle, non-viral vector or viral vector.

If the nuclease of the CRISPR system and the guide RNA are encoded by the same linear DNA product (e.g. the closed linear DNA product) (or by a vector that comprises the linear DNA product (e.g. the closed linear DNA product)) they may be part of the same delivery mechanism. For example, the linear DNA product (e.g. the closed linear DNA product), or the vector, encoding the nuclease of the CRISPR system and the guide RNA may be delivered to a cell by a nanoparticle, a non-viral vector or a viral vector.

The linear DNA product (e.g. the closed linear DNA product) encoding the nuclease of the CRISPR system and the linear DNA product (e.g. the closed linear DNA product) encoding the guide RNA may be delivered to a cell by electroporation. The linear DNA product (e.g. the closed linear DNA product) encoding the nuclease of the CRISPR system and the linear DNA product (e.g. the closed linear DNA product) encoding the guide RNA may be delivered to a cell by hydrodynamic needle.

Thus, the invention provides the linear DNA product (e.g. the closed linear DNA product) described herein for use in CRISPR system delivery to a cell. The invention provides a method of delivering the CRISPR system to a cell, comprising contacting the linear DNA product (e.g. the closed linear DNA product) described herein with a cell. The invention also provides the linear DNA product (e.g. the closed linear DNA product) obtainable by the methods described herein for use in CRISPR system delivery to a cell. The invention provides a method of delivering the CRISPR system to a cell, comprising contacting the linear DNA product (e.g. the closed linear DNA product) obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The linear DNA product produced by the methods described herein may be used in transcription, to generate RNA, preferably mRNA, in vitro or in vivo.

### Kits

The invention provides a kit comprising components required to carry out the method described herein. The kit comprises at least:

(a) first and second adaptor molecules;
(b) a cas nuclease;
(c) at least one guide RNA (gRNA); and
(d) a ligase.

The kit may additionally comprise a DNA polymerase, at least one buffer and/or a nuclease. The buffer may comprise Mg2$^+$.

The first adaptor molecule and/or the second adaptor molecule may comprise a sequence of SEQ ID NO:4 to 15 or a portion thereof. The first adaptor molecule and/or the second adaptor molecule may comprise at least 13, at least 14, at least 15, at least 16, at least 17, at least 18, or at least 19 contiguous nucleotides of SEQ ID NO: 4 to 15. The first and second adapter molecules may comprise an identical nucleic acid sequence. The first and second adapter molecules may comprise a different nucleic acid sequence. The first adaptor molecule may comprise one or more protected nucleotides and the second adaptor molecule may comprise a hairpin or a stem loop region.

The at least one gRNA has a sequence complementary to the at least one sequence in a DNA molecule to use in the method of the invention using the kit.

The first and second adaptor molecule may be provided in a kit together or separately.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The cas nuclease may be cpf1 nuclease or cas9 nuclease.

The ligase may be a DNA ligase, such as a T4 DNA ligase, T7 DNA ligase, mammalian DNA ligase I, III and IV; Taq DNA ligase, Tth DNA ligase, or E. *coli* DNA ligase.

The nuclease may be an exonuclease e.g. exonuclease I, exonuclease III and/or exonuclease VIII.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

### BRIEF DESCRIPTION OF THE DRAWINGS

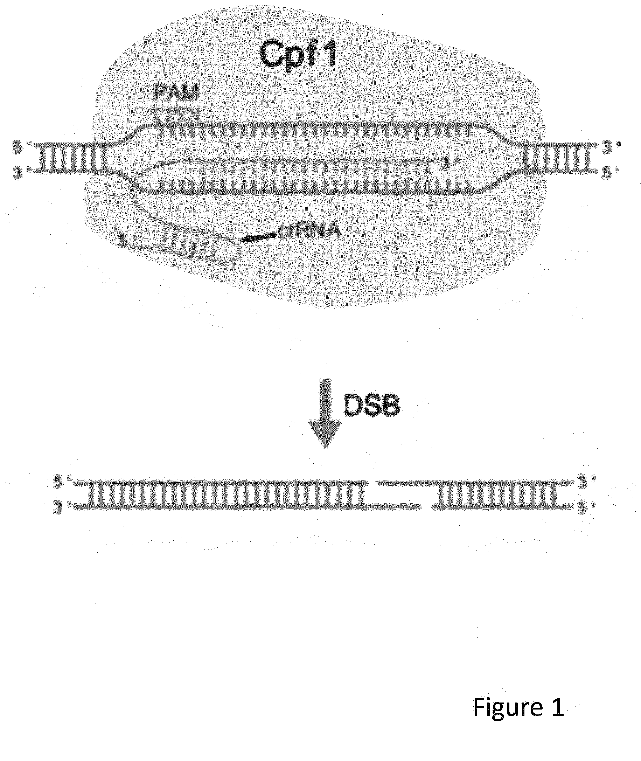

Figure 1 shows a schematic diagram of an RNP complex comprising cpf1 and a guide RNA (gRNA) with a DNA sequence including the PAM sequence 5' of the cut site, and the overhangs that are produced by the cpf1 nuclease.

Figure 2 shows the agarose gel electrophoresis analysis of an in vitro cleavage assay to determine whether a DNA sequence encoding actin could be cleaved by cpf1 using a gRNA complementary to the DNA sequence, as described in Example 2. Lanes 1 to 4 each show the results of assays having slightly different components and ratios, as discussed in Example 2 Lane 5 is amplified DNA without the RNP complex.

Figure 3 shows the results of a combined digestion (cleavage) and ligation reaction of the actin encoding sequence and the RNP comprising cpf1, using the ratios of components shown in Example 3. The reactions were followed by treatment with exonuclease I and exonuclease III to remove any non ligated (i.e. open ended) DNA, and the last 4 lanes show the presence of protected linear DNA.

Figure 4 shows the results of a further set of combined digestion and ligation reactions followed by exonuclease treatment, as described in Example 3.

Figure 5 shows the results of an in vitro cleavage assay using two guide RNAs complementary to an AAV helper encoding sequence. Both guide RNAs are able to direct cleavage (digestion) by cpf1.

Figure 6 shows the results of a combined digestion and ligation reaction using the 2 guide RNAs complementary to the helper sequence, as described in Example 5, to produce closed linear DNA.

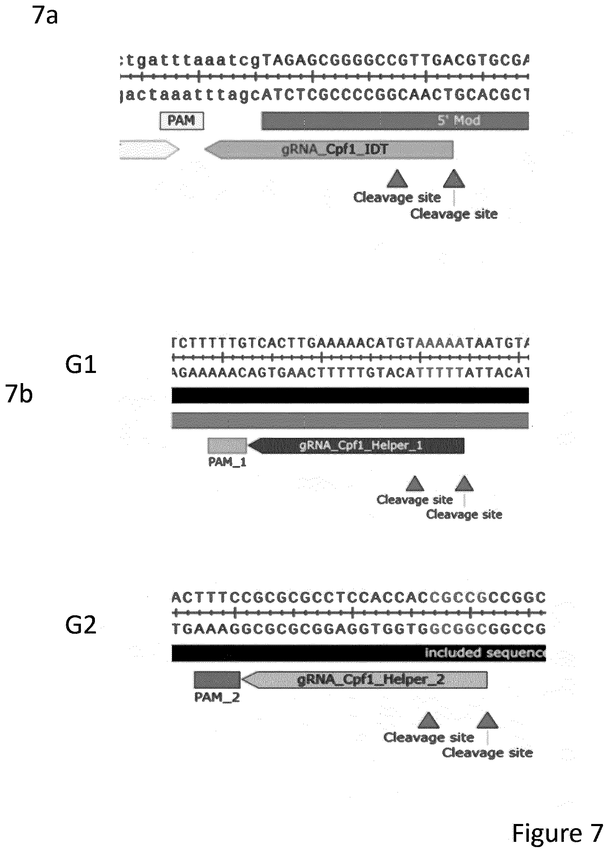

Figure 7 shows schematic diagrams of the different PAM and target sequences used in Examples 2 and 3 (7a) and Examples 4 and 5 (7b).

Figure 8 shows the adaptor molecules with protected nucleotides used in Examples 2 and 3.

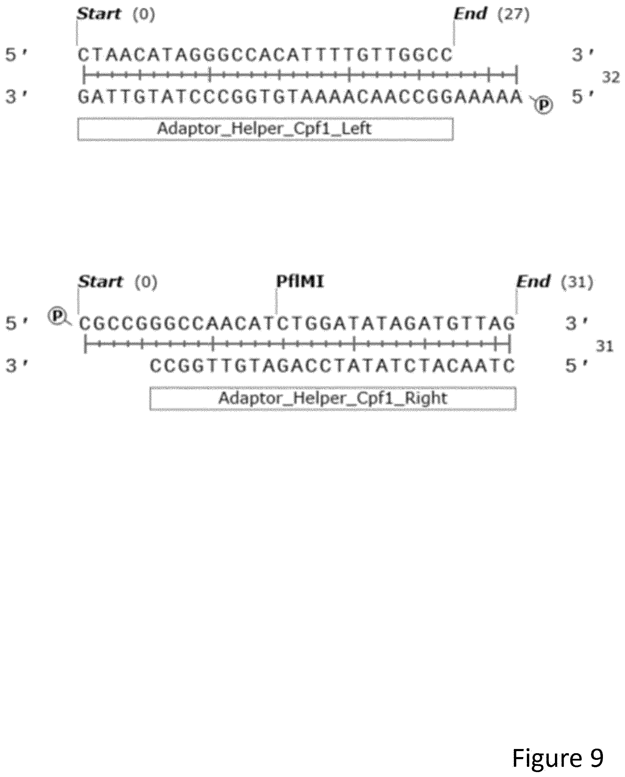

Figure 9 shows the adaptor molecules with protected nucleotides used in Example 4 and 5.

The sequences discussed in the application are provided in the table below:

Table 1. Sequences discussed in the application. * indicates a phosphorothioated nucleotide and r indicates a ribonucleotide.

| SEQ ID NO | name | Sequence |
|---|---|---|
| SEQ ID NO: 1 | guide RNA 1 | /rUrArArUrUrUrCrUrArCrUrCrUrUrGrUrArGrArUrArArUrCrGrUrArGrArGrCrGrGrGrGrCrCrGrUrUrGrA |
| SEQ ID NO: 2 | guide RNA 2 | rUrArArUrUrUrCrUrArCrUrCrUrUrGrUrArGrArUrCrGrCrGrCrGrCrCrUrCrCrArCrCrArCrCrGrCrCrG |
| SEQ ID NO: 3 | guide RNA 3 | rUrArArUrUrUrCrUrArCrUrCrUrUrGrUrArGrArUrUrCrArCrUrUrGrArArArArArCrArUrGrUrArArArArA |
| SEQ ID NO: 4 | adaptor u/s 5OH actin top | GTTGACTAACATTTGT*T*G*G*C*C |
| SEQ ID NO: 5 | adaptor u/s 5OH btm actin | G*G*C*C*A*ACAAATGTTAG |
| SEQ ID NO: 6 | adaptor d/s 5OH top actin | G*G*C*C*A*ACAAATGTTAG |
| SEQ ID NO: 7 | adaptor d/s 5OH btm actin | TCAACCTAACATTTGT*T*G*G*C*C |
| SEQ ID NO: 8 | adaptor u/s 4OH top actin | G*G*C*C*A*ACAAATGTTAGG |
| SEQ ID NO: 9 | adaptor u/s 4OH btm actin | TCAACCTAACATTTGT*T*G*G*C*C |
| SEQ ID NO: 10 | adaptor d/s 4OH top actin | GTTGACTAACATTTGT*T*G*G*C*C |
| SEQ ID NO:11 | adaptor d/s 4OH btm actin | G*G*C*C*A*ACAAATGTTAGT |
| SEQ ID NO:12 | adaptor u/s top helper | CGCCGGGCCAACATCTGGATATAGA*T*G*T*T*AG |
| SEQ ID NO:13 | adaptor u/s btm helper | C*T*A*A*C*ATCTATATCCAGATGTTGGCC |

(continued)

| SEQ ID NO | name | Sequence |
|---|---|---|
| SEQ ID NO:14 | adaptor d/s top helper | C*T*A*A*C*ATAGGGCCACATTTTGTTGGCC |
| SEQ ID NO:15 | adaptor d/s btm helper | AAAAAGGCCAACAAAATGTGGCCCTAT*G*T*T*A*G |

## EXAMPLES

## Example 1: Rolling circle amplification of Cre-derived circular DNA

Cre recombinase from the P1 bacteriophage is a Type I topoisomerase. The enzyme catalyzes the site-specific recombination of DNA between loxP sites. LoxP recognition site (34 bp) consists of two 13 bp inverted repeats which flank an 8 bp spacer region, which confers directionality. The products of Cremediated recombination are dependent upon the location and relative orientation of the loxP sites. Two DNA species containing single loxP sites were fused. DNA found between two loxP sites oriented in the same direction was excised as a circular loop of DNA, while DNA between opposing loxP sites was inverted with respect to external sequences. Cre recombinase requires no additional cofactors or accessory proteins for its function.

Cre reaction conditions: reaction volume 50 μl, DNA of interest purified from agarose gel electrophoresis after restriction enzyme digestion (100 ng), Cre recombinase (NEB, 4 units), incubation time and temperature: 30 min at 37°C and 20 min at 80°C. Next, to remove remaining non-circular DNA molecules before the amplification step, E. *coli* exonuclease I (NEB, 20 units) and III (NEB, 100 units) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like TthPrimPol (Picher *et al,* 2016) that synthesizes the primers for Phi29DNApol during the amplification reaction, or both primers and primase.

Before the amplification, circularized DNA samples were first denatured by adding 1 volume of buffer D (400 mM KOH, 10 mM EDTA) and incubating 3 min at room temperature. Samples were then neutralized by adding 1 volume of buffer N (400 mM HCl, 600 mM Tris-HCl pH 7.5). Rolling circle amplification conditions: 10 ml reaction volume, 1 ml TruePrime WGA reaction buffer 10x (4basebio), 500 μl denatured DNA sample, 1 ml TthPrimPol (1 μM), 160 μl QualiPhi Phi29DNApol (12,5 μM), 2.5 units PPase (Thermo) and 1 ml dNTPs (10 mM). Incubation time and temperature: 20 hours at 30°C and 10 min at 65°C.

## Example 2: In vitro cleavage assay using cpf1 nuclease

Four different in vitro cleavage assays were performed using an Actin DNA sequence and different formulations of the RNP complex using an gRNA having a sequence of SEQ ID NO:1 and cpf1 protein (Alt-R A.s. Cas12a (Cpf1) Ultra obtained from Integrated DNA Technologies, Inc) at the following concentrations and amounts.

| | 1 | 2 | 3 | 4 |
|---|---|---|---|---|
| H20 | 27.32 | 24.32 | 24.64 | 21.64 |
| NEB r3.1 | | 3 | - | 3 |
| gRNA 4.29ng/ul | 1.28 | 1.28 | 2.56 | 2.56 |
| Cpf1 4.7ng/ul | 1.4 | 1.4 | 2.8 | 2.8 |
| Total (ul) | 30 | 30 | 30 | 30 |

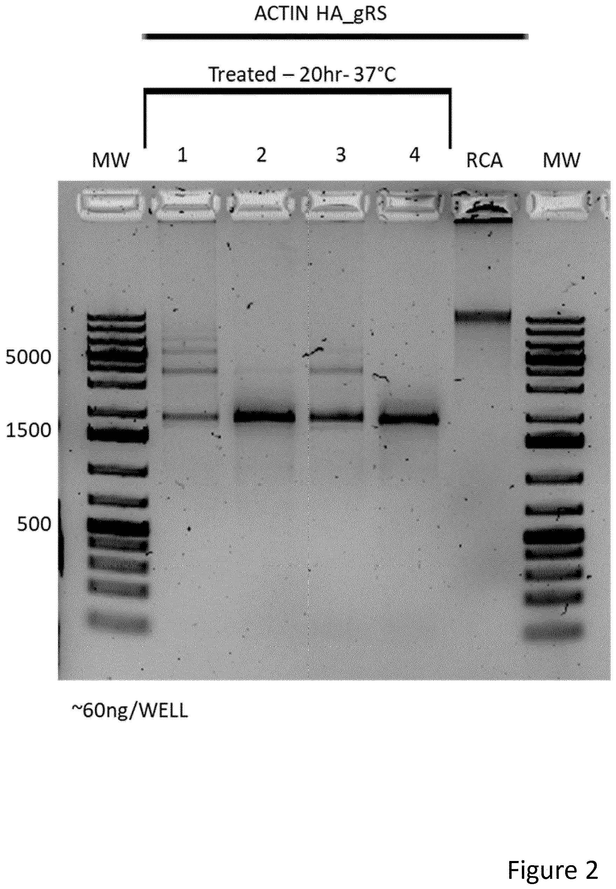

DNA containing the ACTIN DNA sequence and the TTTN PAM sequence for cpf1 was incubated with the RNP complex for 20 hours at 37C to confirm that in vitro cleavage is possible. The concentration of each component was 4.7ng/ul Cpf1; 4.29ng/ul gRNA and 3.15ng/ul of amplified DNA, with the molar ratio being 10:10:1. Fig. 2 shows the results of each of the cleavage reactions, in lanes labelled 1, 2, 3 and 4, showing that the cpf1 nuclease was able to cleave the DNA at the expected sequence to produce the expected fragment of 1.6kb. The non-cleaved DNA is in the lane labelled RCA

**Example 3: digestion and ligation of amplified DNA using cpf1 and T4 ligase**

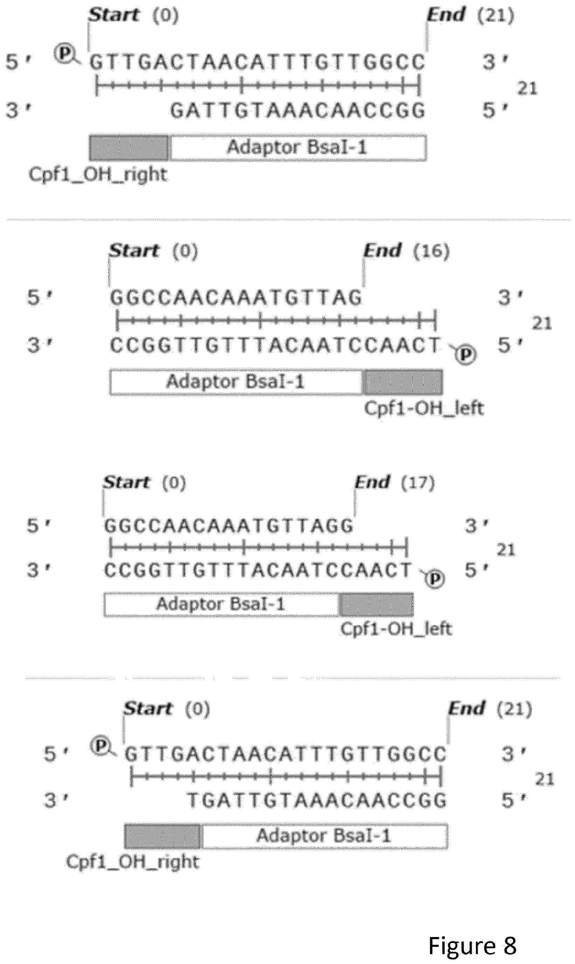

Amplified DNAs were incubated with the RNP complex as described above, T4 DNA ligase and complementary adaptors to the 5' protruding ends (4 nucleotide or 5 nucleotide overhang, due to ambiguity of overhang created by cpf1) generated by cpf1 nuclease digestion on the amplified DNA. The adaptor sequences are shown in SEQ ID NO: 4 and 5 (downstream 5 overhang) SEQ ID NO: 6 and 7 (upstream 5 overhang), SEQ ID NO: 8 and 9 (4OH upstream) and SEQ ID NO: 10 and 11 (4OH downstream); also shown in Figure 8. Digestion and ligation reaction conditions: reaction volume 100 $\mu$l, 5 $\mu$l reaction buffer T4 DNA ligase 10x (NEB), 170 ng DNA, 50 ul RNP (as above), 6.9ngs T4 DNA ligase, 0.92ul each of 3' and 5' DNA adaptor (1:5 molar excess per end), incubation time and temperature: 20 hours 37C. Exonuclease clean-up reaction conditions: 4.4ng of E. *coli* exonuclease I and 8.4ng/ul of *E. coli* exonuclease III were then added to remove remaining adaptors and open DNA molecules. Incubation time and temperature: 30 minutes at 37°C and 20 minutes at 80°C.

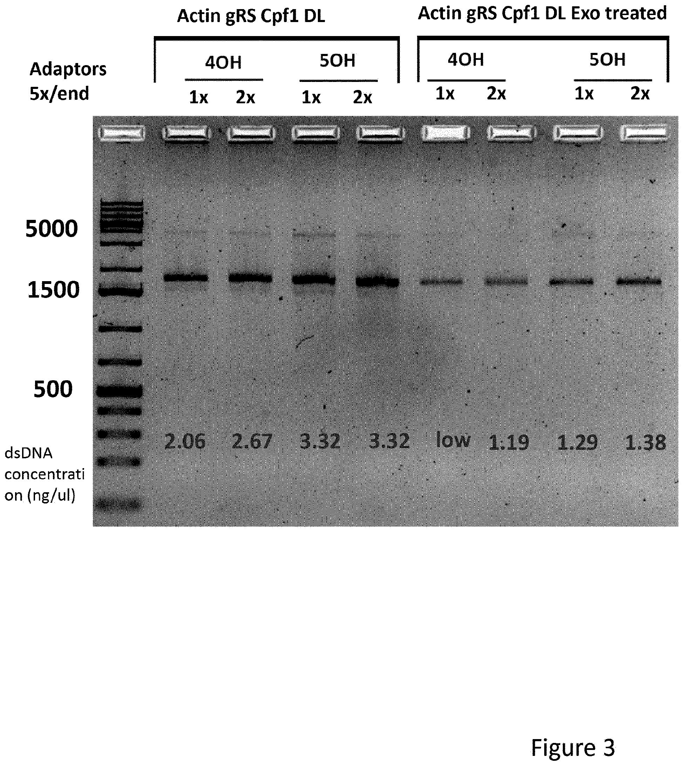

Shown in FIG. 3 is the agarose gel electrophoresis analysis of the amplified DNAs digested with the cpf1 present in the RNP, with T4 ligase and adaptor molecules (lanes 2 to 5). Exonuclease I and III degraded remaining open DNAs and adaptors, resulting in covalently closed linear DNA molecules (lanes 6 to 9) resistant to exonucleolytic degradation.

Ligation yields obtained are shown (Qubit BR dsDNA ng/ul).

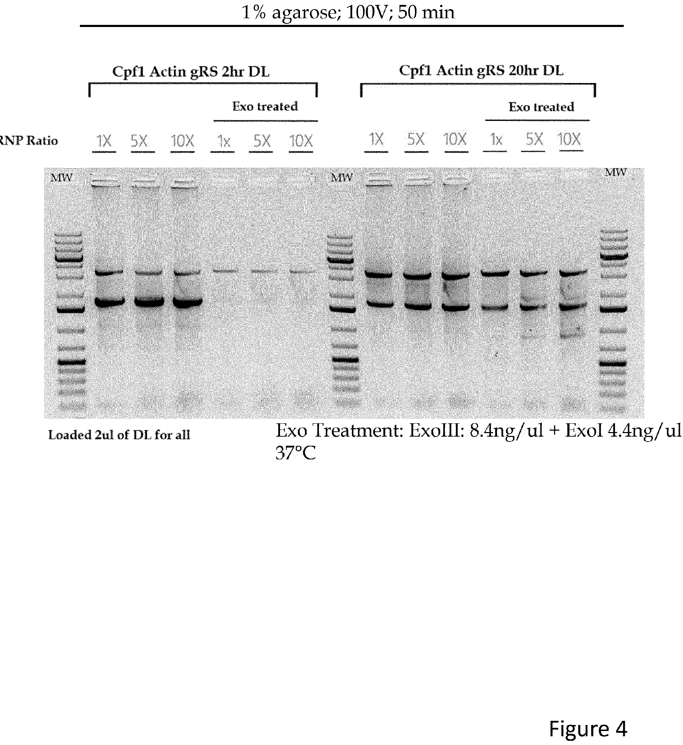

Figure 4 shows further optimisation of the digestion and ligation process, Lane 1 is the molecular weight ladder. Lanes 2 to 4 are the products of a 2 hour digestion/ligation reaction at 37C, at 10:10:1, 5:5:1 and 1:1:1 molar ratio of cpf1:gRNA:amplified DNA, respectively. Lanes 5 to 7 are the same reactions after treatment with ExoI and ExoII, as described above. The expected target band of 3.6kb remains after the breakdown of open DNA and adaptors by the exonuclease treatment. Lane 8 is the molecular weight ladder. Lanes 9 to 11 are the products of a 20 hour digestion/ligation reaction at 37C, at 10:10:1, 5:5:1 and 1:1:1 molar ratio of cpf1:gRNA:amplified DNA, respectively. Lanes 12 to 14 are the same reactions after treatment with ExoI and ExoII, as described above. The expected target band of 3.6kb remains after the breakdown of open DNA and adaptors by the exonuclease treatment. Additional bands are also still present, indicating that some off-target ligation may have occurred.

The DNA concentrations, as measured by Qubit are shown below.

|  | 2hr reaction | | 20 hr reation | |
| --- | --- | --- | --- | --- |
|  | Pre-exo | Post-exo | Pre-exo | Post-exo |
| 1x | 124 | 20.9 | 136 | 81.7 |
| 5x | 144 | 17.8 | 143 | 78.9 |
| 10x | 150 | 18.1 | 136 | 90.7 |

**Example 4: In vitro cleavage assay on a large sequence**

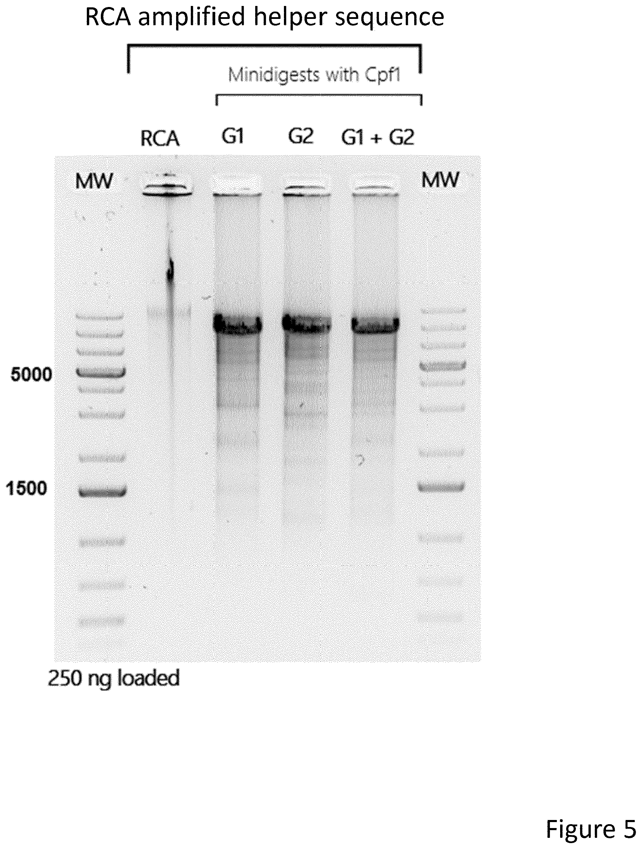

DNA encoding an AAV helper sequence having the TTTN PAM sequence upstream was tested with the cpf1 gRNA RNP complex to determine whether the system is able to cleave larger fragments. Two different guide RNAs were designed (SEQ ID NO: 2 and 3). The RNP complex containing either guide 1, guide 2 or both guide 1 and guide 2 was incubated with the helper sequence for 20 hours at 37C. Figure 5 shows the band of the expected size of 11.2kb when G1, G2 and G1+G2 was used. The molar ratios for the reactions were 5:5:1 cpf1:gRNA:DNA.

**Example 5: production of closed linear DNA encoding an AAV helper sequence, using cpf1 and DNA adaptors.**

A combined digestion (cleavage) and ligation reaction was performed using both G1 and G2 (SEQ ID NO: 2 and 3) as described above, along with DNA adaptors having a 5 nucleotide overhang compatible with the ends of the DNA once cleaved by cpf1. The upstream and downstream adaptor sequences are shown in SEQ ID NO:s 12 to 15, and they have protected nucleotides.

The RNP complex was formed as shown in the table below:

| RNP component | ul |
|---|---|
| Guide 1 | 1.96 |
| Guide 2 | 2.01 |
| NEB buffer r3.1 | 3 |
| Cpf1 | 1.23 |
| H2O | 21.8 |
| Total ul | 30 |

The molar ration of cpf1 :guide RNA (total) to DNA was 5:5:1

The digestion and ligation reaction was carried out with amplified DNA that was purified prior to being added to the digestion and ligation reaction. The components were as follows:

| Components | ul |
|---|---|
| Water | 21.52 |
| 10x T4 ligase buffer | 5.3 |
| ATP | 10 |
| Amplified DNA | 46.15 |
| 5'Adaptor molecule (344.33ng/ul) | 1 |
| 3'adaptor molecule (331.57ng/ul) | 1 |
| T4 ligase (1000ng/ul) | 0.83 |
| RNP | 15 |
| Total (ul) | 100 |

The adaptor molecules were added to a 5x molar excess per end.

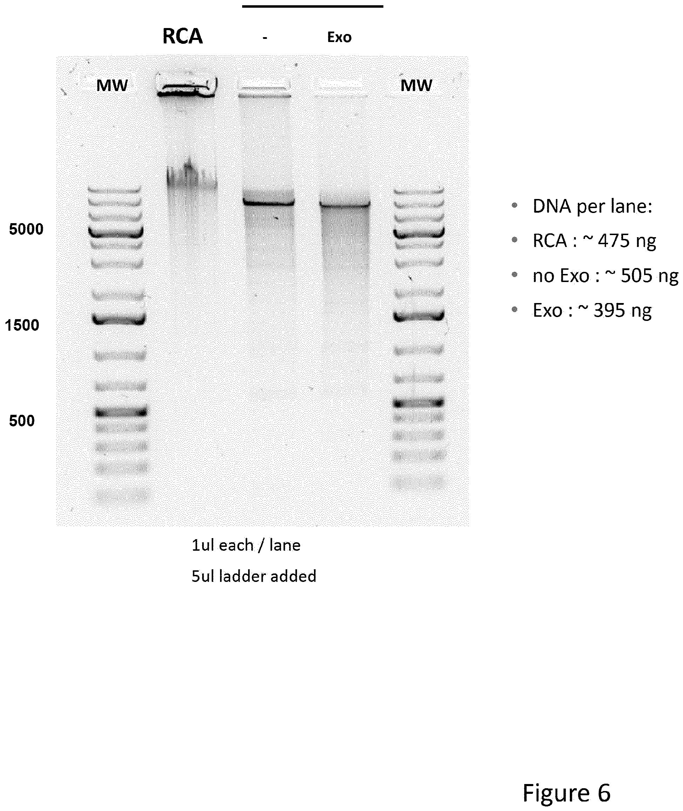

Figure 6 shows the ladder in lanes 1 and 5, the amplified material in lane 2, the material after the digestion and ligation reaction in lane 3, and in lane 4, the material post-exonuclease treatment (as described above). The band in lane 4 is exonuclease resistant, closed ended DNA.

**Claims**

1. A method for producing a linear deoxyribonucleic acid (DNA) product, wherein the method comprises:

(a) contacting a double-stranded DNA molecule comprising at least one target sequence and at least one PAM sequence with an RNP complex comprising a single stranded guide RNA (gRNA) and a cas nuclease to form a digested DNA, wherein the at least one gRNA has a sequence complementary to the at least one target sequence;
(b) contacting the digested DNA with a ligase and first and second adaptor molecules to generate the linear DNA product, wherein the linear DNA product comprises a linear double-stranded region, and wherein the first adaptor molecule is appended to a first end of the linear double-stranded region and the second adaptor molecule is appended to a second end of the linear double-stranded region.

2. A method for producing a linear deoxyribonucleic acid (DNA) product, wherein the method comprises:

(a) contacting a double-stranded DNA molecule comprising at least one target sequence and at least one PAM sequence with an RNP complex comprising a single stranded guide RNA (gRNA) and a cas nuclease, a ligase and first and second adaptor molecules to form a single contiguous aqueous volume, wherein the at least one gRNA has a sequence complementary to the at least one target sequence; and
(b) incubating the single contiguous aqueous volume to generate the linear DNA product, wherein the linear DNA product comprises a linear double-stranded region and wherein the first adaptor molecule is appended to a first end of the linear double-stranded region and the second adaptor molecule is appended to a second end of the linear double-stranded region.

3. The method of claim 1 or claim 2, further comprising the step of forming the RNP complex prior to contacting the double-stranded DNA molecule.

4. The method of any one of claims 1 to 3 wherein the cas nuclease is cpf1.

5. The method of any one of claims 1 to 4 wherein the double-stranded DNA molecule comprises a cassette, which optionally comprises a gene of interest.

6. The method of any one of claims 1 to 5, wherein the double-stranded DNA molecule comprises at least two target sequences and at least two PAM sequences.

7. The method of any one of claims 1 to 6 wherein the at least one target sequence is outside of the cassette.

8. The method of any one of claims 1 to 7 wherein the cas nuclease generates an overhang.

9. The method of any one of claims 1 to 8 wherein the at least one PAM sequence is 5' of the at least one target sequence.

10. The method of any one of claims 1 to 9 wherein the step of contacting the double stranded DNA molecule with an RNP complex is carried out in the presence of $Mg^{2+}$.

11. The method of any one of claims 1 to 10 wherein the first and second adaptor molecules are hairpin adaptor molecules, and the linear DNA product is a closed linear DNA product.

12. The method of any one of claims 1 to 10 wherein the first adaptor molecule is a hairpin adaptor molecule and the second adaptor molecule comprises protected nucleotides, and the linear DNA product is a partially closed DNA product.

13. The method of any one of claims 1 to 10, wherein the first and second adaptor molecules comprise protected nucleotides and the linear DNA product is a protected linear DNA product.

14. A linear DNA product produced by a method of any one of claims 1 to 13.

15. A DNA product produced by a method of any one of claims 1 to 13 for use in medicine.

Figure 1

Figure 2

Figure 3

1% agarose; 100V; 50 min

Cpf1 Actin gRS 2hr DL      Cpf1 Actin gRS 20hr DL

Exo treated      Exo treated

RNP Ratio   1X   5X   10X   1x   5X   10X     1X   5X   10X   1x   5X   10X

Loaded 2ul of DL for all      Exo Treatment: ExoIII: 8.4ng/ul + ExoI 4.4ng/ul 37°C

Figure 4

# RCA amplified helper sequence

Figure 5

- DNA per lane:
- RCA : ~ 475 ng
- no Exo : ~ 505 ng
- Exo : ~ 395 ng

Figure 6

7a

G1

7b

G2

Figure 7

Figure 8

**Start** (0)                                    **End** (27)

5' CTAACATAGGGCCACATTTTGTTGGCC 3'

3' GATTGTATCCCGGTGTAAAACAACCGGAAAAA ℗ 5'    32

Adaptor_Helper_Cpf1_Left

**Start** (0)              **PflMI**              **End** (31)

5' ℗ CGCCGGGCCAACATCTGGATATAGATGTTAG 3'

3'      CCGGTTGTAGACCTATATCTACAATC 5'    31

Adaptor_Helper_Cpf1_Right

Figure 9

Europäisches Patentamt

European Patent Office

Office européen des brevets

# EUROPEAN SEARCH REPORT

**Application Number**

EP 23 20 8666

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/127661 A1 (LI MO [SA] ET AL) 28 April 2022 (2022-04-28) | 1,3-5,8, 11-14 | INV. C12N9/22 |
| Y | * par.13, 54-56, 89-92,94 * | 6,7,9 | C12N15/10 C12N15/113 |
| X | WO 2019/030306 A1 (DEPIXUS [FR]) 14 February 2019 (2019-02-14) | 1-4,8, 10-14 | C12N15/63 C12N15/64 |
| Y | * p.2 l.11, p.25 l.1 – 22, p.3 l.5-23, p.3 l.24 – p.4 l.3, p.25 l.1 – 22, p.9 l.22, p.2 l.28-31, p.35 l.14-30, p.72 l.10, p.2 l.28-31, p.52 l.27 * | 6,7,9 | C12Q1/00 C12Q1/6806 |
| X | US 2023/167441 A1 (ZHONG MINGHONG [US]) 1 June 2023 (2023-06-01) | 1-3,5, 13-15 | |
| Y | * par.3, 17, 31,187 * | 6,7,9 | |
| X | WO 2022/112316 A1 (KEYGENE NV [NL]) 2 June 2022 (2022-06-02) | 1,3,4,8, 14 | |
| Y | * p.1 l.15-19, p.2 l.7 – 9, p.14 l.9, p.21 l.19 * | 6,7,9 | |
| A | US 2023/332256 A1 (HA TAEKJIP [US] ET AL) 19 October 2023 (2023-10-19) * par.7, 95 * | 1-15 | **TECHNICAL FIELDS SEARCHED (IPC)** C12N C12Q G01N C40B |

The present search report has been drawn up for all claims

| Place of search | Date of completion of the search | Examiner |
|---|---|---|
| Munich | 5 April 2024 | Bonello, Steve |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 8666**

This annex lists the patent family members relating to the patent documents cited in the above-mentioned European search report.
The members are as contained in the European Patent Office EDP file on
The European Patent Office is in no way liable for these particulars which are merely given for the purpose of information.

**05-04-2024**

| Patent document cited in search report | | | Publication date | Patent family member(s) | | Publication date |
|---|---|---|---|---|---|---|
| US 2022127661 | A1 | | 28-04-2022 | EP 3935184 | A1 | 12-01-2022 |
| | | | | US 2022127661 | A1 | 28-04-2022 |
| | | | | WO 2020178759 | A1 | 10-09-2020 |
| WO 2019030306 | A1 | | 14-02-2019 | EP 3625356 | A1 | 25-03-2020 |
| | | | | EP 3950957 | A1 | 09-02-2022 |
| | | | | US 2020181683 | A1 | 11-06-2020 |
| | | | | US 2022372548 | A1 | 24-11-2022 |
| | | | | WO 2019030306 | A1 | 14-02-2019 |
| US 2023167441 | A1 | | 01-06-2023 | US 2023167441 | A1 | 01-06-2023 |
| | | | | WO 2023101993 | A2 | 08-06-2023 |
| WO 2022112316 | A1 | | 02-06-2022 | EP 4251761 | A1 | 04-10-2023 |
| | | | | US 2024002904 | A1 | 04-01-2024 |
| | | | | WO 2022112316 | A1 | 02-06-2022 |
| US 2023332256 | A1 | | 19-10-2023 | US 2023332256 | A1 | 19-10-2023 |
| | | | | WO 2022061172 | A2 | 24-03-2022 |

EPO FORM P0459

For more details about this annex : see Official Journal of the European Patent Office, No. 12/82

**REFERENCES CITED IN THE DESCRIPTION**

**Patent documents cited in the description**

- WO 2010086626 A1
- WO 2022229460 A
- WO 2023006978 A